# EUROPEAN PATENT SPECIFICATION

(11) **EP 3 146 073 B1**
(45) Date of publication and mention of the grant of the patent: **24.04.2019**
(21) Application number: 15732388.2
(22) Date of filing: 21.05.2015
(51) Int. Cl.: C12Q 1/6883, C12Q 1/6886

(54) **A DIAGNOSTIC MARKER FOR PAGET DISEASE**
DIAGNOSTISCHER MARKER FÜR MORBUS PAGET
MARQUEUR DE DIAGNOSTIC POUR LA MALADIE DE PAGET

(30) Priority: 21.05.2014 IT TO20140404
(43) Date of publication of application: 29.03.2017
(73) Proprietor: Consiglio Nazionale Delle Ricerche, 00185 Roma (IT)
(72) Inventor: GIANFRANCESCO, Fernando, I-81014 Fontegreca (Caserta) (IT)
(74) Representative: Comoglio, Elena
(86) International application number: PCT/IB2015/053731
(87) International publication number: WO 2015/177744

(56) References cited:
- EP-A1- 1 559 781
- EP-A2- 1 074 617
- WO-A2-02/068579
- WO-A2-03/012134
- FERNANDO GIANFRANCESCO ET AL: "Giant cell tumor occurring in familial Paget's disease of bone: Report of clinical characteristics and linkage analysis of a large pedigree", JOURNAL OF BONE AND MINERAL RESEARCH, vol. 28, no. 2, 15 January 2013 (2013-01-15), pages 341-350, XP055166659, ISSN: 0884-0431, DOI: 10.1002/jbmr.1750 cited in the application

### SEQUENCE LISTING

<110> Consiglio Nazionale delle Ricerche
<120> Diagnostic marker for Paget's disease of bone
<130> E0098531-EC
<160> 30
<170> PatentIn version 3.5
<210> 1
   <211> 3714
   <212> DNA
   <213> Homo sapiens
<400> 1
<210> 2
   <211> 3714
   <212> DNA
   <213> Homo sapiens
<400> 2
<210> 3
   <211> 3714
   <212> DNA
   <213> Homo sapiens
<400> 3
<210> 4
   <211> 3714
   <212> DNA
   <213> Homo sapiens
<400> 4
   atgggggata tgaagacccc tgattttgat gacctccttg ctgcctttga catccctgac 60
   attgatgcga atgaagccat ccattctggg ccagaagaaa atgaggggcc tggaggccca 120
   gggaagccag aaccaggtgt aggaagtgaa tctgaagaca cagcagcagc ctctgctggg 180
   gatggccctg gagttccagc ccaggcctct gaccatggcc tgccaccgcc agacatttct 240
   gtagtcagtg tcattgtcaa gaacactgtg tgtcccgagc agtctgaggc cctggctgga 300
   ggctcagcag gagacggggc ccaggctgct ggggtaacta aagaagggcc tgtggggcct 360
   catcgaatgc agaatggttt tgggagccct gaaccttccc tcccaggaac tccccactct 420
   cctgctcctc ccagtggggg cacctggaaa gaaaaaggca tggaaggcaa aactcccttg 480
   gacctgtttg ctcattttgg ccctgagcca ggggaccact cagatccgct gcctccctct 540
   gcaccctctc ccactcggga gggggctctg accccgcctc ctttcccctc ttcctttgag 600
   ctggcccagg agaatggccc aggcatgcag ccacctgttt cttccccacc attgggggcc 660
   ttgaagcagg agagctgcag cccccatcat ccccaggtcc tagcccaaca aggctcaggc 720
   tccagcccta aggccacgga catccctgcc agtgcctcgc ctcccccagt tgctggggtg 780
   cccttcttca agcagtctcc agggcaccag agccctcttg cctcccccaa agtgcccgtc 840
   tgtcagccct tgaaggaaga agatgatgat gaggggccag tggacaagtc ttccccagga 900
   agtccccaga gtccctctag tggggccgag gctgcagatg aggacagcaa tgactcccct 960
   gcctccagct cctctaggcc tcttaaggtg cggatcaaga ccattaaaac atcctgcggg    1020
   aatatcacaa ggactgtaac tcaggtcccc tcagatcctg atccacctgc ccccttggct    1080
   gagggggcct tcttggctga ggctagcctc ttgaagctgt cccctgcaac acctacttct 1140
   gagggtccaa aggtggtgag cgtacagttg ggtgatggta caaggctgaa aggcactgtg 1200
   ctgcctgtgg ccaccatcca gaacgccagt actgccatgc tgatggcagc cagtgtggct 1260
   cgcaaggctg tggtgctgcc tggggggact gccaccagcc ctaagatgat tgctaagaac 1320
   gtgctaggcc tggtgcccca agccctgcct aaggctgacg ggcgggcagg gctggggact 1380
   gggggacaga aggtgaatgg tgcctcggtg gtgatggtgc aaccttcaaa gacagctact 1440
   gggccaagta cagggggcgg cacagtgata tcacggaccc agtccagcct ggtggaggcc 1500
   ttcaacaaga tcctcaacag caagaacctg ctccctgcct ataggccaaa cctgagccca 1560
   ccagctgagg ctgggctggc cctgcctccc accggctacc gctgcctgga gtgtggggat 1620
   gccttctcat tggagaagag cctggcacgg cactatgacc gtcggagcat gcgcatcgag 1680
   gtcacctgca accactgcgc ccgccgcctg gtcttcttca acaagtgcag cctgctcctg 1740
   catgcacgtg aacacaagga caaggggctc gtcatgcagt gctcacattt ggtcatgagg 1800
   cctgtagccc ttgaccagat ggtggggcag ccggacatca caccgctgct gcctgtagct 1860
   gtcccacctg tctctggacc tctggccttg cctgccttgg gcaagggtga gggggccatc 1920
   acctcctctg ccattactac agttgctgct gaggcccctg tcctgccgct ctccacagag 1980
   ccgcctgctg ccccggccac ctctgcttac acatgctttc gctgcctgga gtgcaaggaa 2040
   cagtgccggg acaaggctgg catggcagct cacttccagc agctcggccc ccctgcccct 2100
   ggggccacca gcaatgtgtg cccaacctgc cccatgatgc tccccaatcg ctgcagcttc 2160
   agcgcccacc agcgcatgca taagaatcga cccccccatg tctgtcctga gtgtgggggc 2220
   aacttcctgc aagccaattt tcagacccat ctccgggagg cctgtctgca cgtctctcgc 2280
   cgtgtaggat acaggtgccc cagctgttca gtggtgtttg ggggtgtgaa ctccatcaag 2340
   tcccacatcc agacgtcgca ctgcgaggtt ttccacaagt gccccatctg ccccatggcc 2400
   ttcaagtctg ggccaagtgc ccatgcccac ctctactccc agcatcccag cttccaaact 2460
   cagcaggcca agctgatcta caagtgcgcc atgtgcgaca cagtcttcac tcacaaaccc 2520
   ctcctctcct cacacttcga ccagcacttg ctgccccagc gtgtcagtgt ctttaagtgc 2580
   ccgtcttgtc ctctgctctt tgcccaaaaa aggaccatgc tggaacatct caagaacacc 2640
   catcagtctg ggcgcttgga ggagactgct gggaaagggg ccgggggtgc cctgctgacc 2700
   cccaagactg agcctgagga gctggctgtt tctcagggag gggcagcccc tgctactgag 2760
   gagtcgtctt catcttcaga agaggaggaa gtacccagct cccctgagcg cccccgtcca 2820
   gccaaacggc ctcggcggga actagggagc aaaggcctca agggtggggg tggggggcct   2880
   ggaggctgga cctgtggcct gtgtcactcc tggttccctg agcgtgatga atacgtggcc   2940
   cacatgaaga aggagcatgg caagtcagtg aaaaagttcc cctgtcgcct gtgtgagcgc   3000
   tccttctgct ccgcccccag cctgaggcgc catgtcagag ttaatcacga gggcatcaag   3060
   cgagtttacc cctgcaggta ttgcacagag ggaaaacgca ccttcagcag ccgcctgatc   3120
   ctagagaaac atgtccaggt ccggcacggc ttgcagcttg gggcccagtc ccctggccgg    3180
   gggaccacct tggctcgggg ttccagtgcc agagcccagg ggccaggtcg gaaacgccgc   3240
   cagtcttctg actcttgcag tgaggagcct gacagcacga caccgccagc caagtccccc   3300
   aggggcggac ctggatctgg aggccatggc cctctgcgct accggagcag cagctccaca   3360
   gaacagagcc tcatgatggg gttgagggtg gaggatggtg cccagcagtg cctcgactgt   3420
   ggcttgtgct ttgcctcccc tggctccctg agccgacacc gtttcatcag ccacaagaag   3480
   agacggggtg tgggtaaagc cagtgccctg gggctggggg atggggagga agaggcccct    3540
   ccatcaaggt ctgaccccga tggtggagac tcacccctgc ctgcttctgg aggcccactg   3600
   acctgtaagg tctgtggcaa gagctgcgac agccctctaa acctcaagac ccacttccgc   3660
   acgcatggca tggcgttcat cagggctcgg cagggggctg ttggggacaa ctag          3714
<210> 5
   <211> 1237
   <212> PRT
   <213> Homo sapiens
<400> 5
   Met Gly Asp Met Lys Thr Pro Asp Phe Asp Asp Leu Leu Ala Ala Phe
   1               5                   10                  15
   Asp Ile Pro Asp Ile Asp Ala Asn Glu Ala Ile His Ser Gly Pro Glu
               20                  25                  30
   Glu Asn Glu Gly Pro Gly Gly Pro Gly Lys Pro Glu Pro Gly Val Gly
           35                  40                  45
   Ser Glu Ser Glu Asp Thr Ala Ala Ala Ser Ala Gly Asp Gly Pro Gly
       50                  55                  60
   Val Pro Ala Gln Ala Ser Asp His Gly Leu Pro Pro Pro Asp Ile Ser
   65                  70                  75                  80
   Val Val Ser Val Ile Val Lys Asn Thr Val Cys Pro Glu Gln Ser Glu
                   85                  90                  95
   Ala Leu Ala Gly Gly Ser Ala Gly Asp Gly Ala Gln Ala Ala Gly Val
               100                 105                 110
   Thr Lys Glu Gly Pro Val Gly Pro His Arg Met Gln Asn Gly Phe Gly
           115                 120                 125
   Ser Pro Glu Pro Ser Leu Pro Gly Thr Pro His Ser Pro Ala Pro Pro
       130                 135                 140
   Ser Gly Gly Thr Trp Lys Glu Lys Gly Met Glu Gly Lys Thr Pro Leu
   145                 150                 155                 160
   Asp Leu Phe Ala His Phe Gly Pro Glu Pro Gly Asp His Ser Asp Pro
                   165                 170                 175
   Leu Pro Pro Ser Ala Pro Ser Pro Thr Arg Glu Gly Ala Leu Thr Pro
               180                 185                 190
   Pro Pro Phe Pro Ser Ser Phe Glu Leu Ala Gln Glu Asn Gly Pro Gly
           195                 200                 205
   Met Gln Pro Pro Val Ser Ser Pro Pro Leu Gly Ala Leu Lys Gln Glu
       210                 215                 220
   Ser Cys Ser Pro His His Pro Gln Val Leu Ala Gln Gln Gly Ser Gly
   225                 230                 235                 240
   Ser Ile Pro Lys Ala Thr Asp Ile Pro Ala Ser Ala Ser Pro Pro Pro
                   245                 250                 255
   Val Ala Gly Val Pro Phe Phe Lys Gln Ser Pro Gly His Gln Ser Pro
               260                 265                 270
   Leu Ala Ser Pro Lys Val Pro Val Cys Gln Pro Leu Lys Glu Glu Asp
           275                 280                 285
   Asp Asp Glu Gly Pro Val Asp Lys Ser Ser Pro Gly Ser Pro Gln Ser
       290                 295                 300
   Pro Ser Ser Gly Ala Glu Ala Ala Asp Glu Asp Ser Asn Asp Ser Pro
   305                 310                 315                 320
   Ala Ser Ser Ser Ser Arg Pro Leu Lys Val Arg Ile Lys Thr Ile Lys
                   325                 330                 335
   Thr Ser Cys Gly Asn Ile Thr Arg Thr Val Thr Gln Val Pro Ser Asp
               340                 345                 350
   Pro Asp Pro Pro Ala Pro Leu Ala Glu Gly Ala Phe Leu Ala Glu Ala
           355                 360                 365
   Ser Leu Leu Lys Leu Ser Pro Ala Thr Pro Thr Ser Glu Gly Pro Lys
       370                 375                 380
   Val Val Ser Val Gln Leu Gly Asp Gly Thr Arg Leu Lys Gly Thr Val
   385                 390                 395                 400
   Leu Pro Val Ala Thr Ile Gln Asn Ala Ser Thr Ala Met Leu Met Ala
                   405                 410                 415
   Ala Ser Val Ala Arg Lys Ala Val Val Leu Pro Gly Gly Thr Ala Thr
               420                 425                 430
   Ser Pro Lys Met Ile Ala Lys Asn Val Leu Gly Leu Val Pro Gln Ala
           435                 440                 445
   Leu Pro Lys Ala Asp Gly Arg Ala Gly Leu Gly Thr Gly Gly Gln Lys
       450                 455                 460
   Val Asn Gly Ala Ser Val Val Met Val Gln Pro Ser Lys Thr Ala Thr
   465                 470                 475                 480
   Gly Pro Ser Thr Gly Gly Gly Thr Val Ile Ser Arg Thr Gln Ser Ser
                   485                 490                 495
   Leu Val Glu Ala Phe Asn Lys Ile Leu Asn Ser Lys Asn Leu Leu Pro
               500                 505                 510
   Ala Tyr Arg Pro Asn Leu Ser Pro Pro Ala Glu Ala Gly Leu Ala Leu
           515                 520                 525
   Pro Pro Thr Gly Tyr Arg Cys Leu Glu Cys Gly Asp Ala Phe Ser Leu
       530                 535                 540
   Glu Lys Ser Leu Ala Arg His Tyr Asp Arg Arg Ser Met Arg Ile Glu
   545                 550                 555                 560
   Val Thr Cys Asn His Cys Ala Arg Arg Leu Val Phe Phe Asn Lys Cys
                   565                 570                 575
   Ser Leu Leu Leu His Ala Arg Glu His Lys Asp Lys Gly Leu Val Met
               580                 585                 590
   Gln Cys Ser His Leu Val Met Arg Pro Val Ala Leu Asp Gln Met Val
           595                 600                 605
   Gly Gln Pro Asp Ile Thr Pro Leu Leu Pro Val Ala Val Pro Pro Val
       610                 615                 620
   Ser Gly Pro Leu Ala Leu Pro Ala Leu Gly Lys Gly Glu Gly Ala Ile
   625                 630                 635                 640
   Thr Ser Ser Ala Ile Thr Thr Val Ala Ala Glu Ala Pro Val Leu Pro
                   645                 650                 655
   Leu Ser Thr Glu Pro Pro Ala Ala Pro Ala Thr Ser Ala Tyr Thr Cys
               660                 665                 670
   Phe Arg Cys Leu Glu Cys Lys Glu Gln Cys Arg Asp Lys Ala Gly Met
           675                 680                 685
   Ala Ala His Phe Gln Gln Leu Gly Pro Pro Ala Pro Gly Ala Thr Ser
       690                 695                 700
   Asn Val Cys Pro Thr Cys Pro Met Met Leu Pro Asn Arg Cys Ser Phe
   705                 710                 715                 720
   Ser Ala His Gln Arg Met His Lys Asn Arg Pro Pro His Val Cys Pro
                   725                 730                 735
   Glu Cys Gly Gly Asn Phe Leu Gln Ala Asn Phe Gln Thr His Leu Arg
               740                 745                 750
   Glu Ala Cys Leu His Val Ser Arg Arg Val Gly Tyr Arg Cys Pro Ser
           755                 760                 765
   Cys Ser Val Val Phe Gly Gly Val Asn Ser Ile Lys Ser His Ile Gln
       770                 775                 780
   Thr Ser His Cys Glu Val Phe His Lys Cys Pro Ile Cys Pro Met Ala
   785                 790                 795                 800
   Phe Lys Ser Gly Pro Ser Ala His Ala His Leu Tyr Ser Gln His Pro
                   805                 810                 815
   Ser Phe Gln Thr Gln Gln Ala Lys Leu Ile Tyr Lys Cys Ala Met Cys
               820                 825                 830
   Asp Thr Val Phe Thr His Lys Pro Leu Leu Ser Ser His Phe Asp Gln
           835                 840                 845
   His Leu Leu Pro Gln Arg Val Ser Val Phe Lys Cys Pro Ser Cys Pro
   850 855 860
   Leu Leu Phe Ala Gln Lys Arg Thr Met Leu Glu His Leu Lys Asn Thr
   865 870 875 880
   His Gln Ser Gly Arg Leu Glu Glu Thr Ala Gly Lys Gly Ala Gly Gly
   885 890 895
   Ala Leu Leu Thr Pro Lys Thr Glu Pro Glu Glu Leu Ala Val Ser Gln
   900 905 910
   Gly Gly Ala Ala Pro Ala Thr Glu Glu Ser Ser Ser Ser Ser Glu Glu
   915 920 925
   Glu Glu Val Pro Ser Ser Pro Glu Pro Pro Arg Pro Ala Lys Arg Pro
   930 935 940
   Arg Arg Glu Leu Gly Ser Lys Gly Leu Lys Gly Gly Gly Gly Gly Pro
   945 950 955 960
   Gly Gly Trp Thr Cys Gly Leu Cys His Ser Trp Phe Pro Glu Arg Asp
   965 970 975
   Glu Tyr Val Ala His Met Lys Lys Glu His Gly Lys Ser Val Lys Lys
   980 985 990
   Phe Pro Cys Arg Leu Cys Glu Arg Ser Phe Cys Ser Ala Pro Ser Leu
   995 1000 1005
   Arg Arg His Val Arg Val Asn His Glu Gly Ile Lys Arg Val Tyr
   1010 1015 1020
   Pro Cys Arg Tyr Cys Thr Glu Gly Lys Arg Thr Phe Ser Ser Arg
   1025 1030 1035
   Leu Ile Leu Glu Lys His Val Gln Val Arg His Gly Leu Gln Leu
   1040 1045 1050
   Gly Ala Gln Ser Pro Gly Arg Gly Thr Thr Leu Ala Arg Gly Ser
   1055 1060 1065
   Ser Ala Arg Ala Gln Gly Pro Gly Arg Lys Arg Arg Gln Ser Ser
   1070 1075 1080
   Asp Ser Cys Ser Glu Glu Pro Asp Ser Thr Thr Pro Pro Ala Lys
   1085 1090 1095
   Ser Pro Arg Gly Gly Pro Gly Ser Gly Gly His Gly Pro Leu Arg
       1100                1105                1110
   Tyr Arg Ser Ser Ser Ser Thr Glu Gln Ser Leu Met Met Gly Leu
       1115                1120                1125
   Arg Val Glu Asp Gly Ala Gln Gln Cys Leu Asp Cys Gly Leu Cys
       1130                1135                1140
   Phe Ala Ser Pro Gly Ser Leu Ser Arg His Arg Phe Ile Ser His
       1145                1150                1155
   Lys Lys Arg Arg Gly Val Gly Lys Ala Ser Ala Leu Gly Leu Gly
       1160                1165                1170
   Asp Gly Glu Glu Glu Ala Pro Pro Ser Arg Ser Asp Pro Asp Gly
       1175                1180                1185
   Gly Asp Ser Pro Leu Pro Ala Ser Gly Gly Pro Leu Thr Cys Lys
       1190                1195                1200
   Val Cys Gly Lys Ser Cys Asp Ser Pro Leu Asn Leu Lys Thr His
       1205                1210                1215
   Phe Arg Thr His Gly Met Ala Phe Ile Arg Ala Arg Gln Gly Ala
       1220                1225                1230
   Val Gly Asp Asn
       1235
<210> 6
   <211> 1237
   <212> PRT
   <213> Homo sapiens
<400> 6
   Met Gly Asp Met Lys Thr Pro Asp Phe Asp Asp Leu Leu Ala Ala Phe
   1               5                   10                  15
   Asp Ile Pro Asp Ile Asp Ala Asn Glu Ala Ile His Ser Gly Pro Glu
               20                  25                  30
   Glu Asn Glu Gly Pro Gly Gly Pro Gly Lys Pro Glu Pro Gly Val Gly
           35                  40                  45
   Ser Glu Ser Glu Asp Thr Ala Ala Ala Ser Ala Gly Asp Gly Pro Gly
       50                  55                  60
   Val Pro Ala Gln Ala Ser Asp His Gly Leu Pro Pro Pro Asp Ile Ser
   65                  70                  75                  80
   Val Val Ser Val Ile Val Lys Asn Thr Val Cys Pro Glu Gln Ser Glu
                   85                  90                  95
   Ala Leu Ala Gly Gly Ser Ala Gly Asp Gly Ala Gln Ala Ala Gly Val
               100                 105                 110
   Thr Lys Glu Gly Pro Val Gly Pro His Arg Met Gln Asn Gly Phe Gly
           115                 120                 125
   Ser Pro Glu Pro Ser Leu Pro Gly Thr Pro His Ser Pro Ala Pro Pro
       130                 135                 140
   Ser Gly Gly Thr Trp Lys Glu Lys Gly Met Glu Gly Lys Thr Pro Leu
   145                 150                 155                 160
   Asp Leu Phe Ala His Phe Gly Pro Glu Pro Gly Asp His Ser Asp Pro
                   165                 170                 175
   Leu Pro Pro Ser Ala Pro Ser Pro Thr Arg Glu Gly Ala Leu Thr Pro
               180                 185                 190
   Pro Pro Phe Pro Ser Ser Phe Glu Leu Ala Gln Glu Asn Gly Pro Gly
           195                 200                 205
   Met Gln Pro Pro Val Ser Ser Pro Pro Leu Gly Ala Leu Lys Gln Glu
       210                 215                 220
   Ser Cys Ser Pro His His Pro Gln Val Leu Ala Gln Gln Gly Ser Gly
   225                 230                 235                 240
   Ser Ser Pro Lys Ala Thr Asp Ile Pro Ala Ser Ala Ser Pro Pro Pro
                   245                 250                 255
   Val Ala Gly Val Pro Phe Phe Lys Gln Ser Pro Gly His Gln Ser Pro
               260                 265                 270
   Leu Ala Ser Pro Lys Val Pro Val Cys Gln Pro Leu Lys Glu Glu Asp
           275                 280                 285
   Asp Asp Glu Gly Pro Val Asp Lys Ser Ser Pro Gly Ser Pro Gln Ser
       290                 295                 300
   Pro Ser Ser Gly Ala Glu Ala Ala Asp Glu Asp Ser Asn Asp Ser Pro
   305                 310                 315                 320
   Ala Ser Ser Ser Ser Arg Pro Leu Lys Val Arg Ile Lys Thr Ile Lys
                   325                 330                 335
   Thr Ser Cys Gly Asn Ile Thr Arg Thr Val Thr Gln Val Pro Ser Asp
               340                 345                 350
   Pro Asp Pro Pro Ala Pro Leu Ala Glu Gly Ala Phe Leu Ala Glu Ala
           355                 360                 365
   Ser Leu Leu Lys Leu Ser Pro Ala Thr Pro Thr Ser Glu Gly Pro Lys
       370                 375                 380
   Val Val Ser Val Gln Leu Gly Asp Gly Thr Arg Leu Lys Gly Thr Val
   385                 390                 395                 400
   Leu Pro Val Ala Thr Ile Gln Asn Ala Ser Thr Ala Met Leu Met Ala
                   405                 410                 415
   Ala Ser Val Ala Arg Lys Ala Val Val Leu Pro Gly Gly Thr Ala Thr
               420                 425                 430
   Ser Pro Lys Met Ile Ala Lys Asn Val Leu Gly Leu Val Pro Gln Ala
           435                 440                 445
   Leu Pro Lys Ala Asp Gly Arg Ala Gly Leu Gly Thr Gly Gly Gln Lys
       450                 455                 460
   Val Asn Gly Ala Ser Val Val Met Val Gln Pro Ser Lys Thr Ala Thr
   465                 470                 475                 480
   Gly Pro Ser Thr Gly Gly Gly Thr Val Ile Ser Arg Thr Gln Ser Ser
                   485                 490                 495
   Leu Val Glu Ala Phe Asn Lys Ile Leu Asn Ser Lys Asn Leu Leu Pro
               500                 505                 510
   Ala Tyr Arg Pro Asn Leu Ser Pro Pro Ala Glu Ala Gly Leu Ala Leu
           515                 520                 525
   Pro Pro Thr Gly Tyr Arg Cys Leu Glu Cys Gly Asp Ala Phe Ser Leu
       530                 535                 540
   Glu Lys Ser Leu Ala Arg His Tyr Asp Arg Arg Ser Met Arg Ile Glu
   545                 550                 555                 560
   Val Thr Cys Asn His Cys Ala Arg Arg Leu Val Phe Phe Asn Lys Cys
                   565                 570                 575
   Ser Leu Leu Leu His Ala Arg Glu His Lys Asp Lys Gly Leu Val Met
               580                 585                 590
   Gln Cys Ser His Leu Val Met Arg Pro Val Ala Leu Asp Gln Met Val
           595                 600                 605
   Gly Gln Pro Asp Ile Thr Pro Leu Leu Pro Val Ala Val Pro Pro Val
       610                 615                 620
   Ser Gly Pro Leu Ala Leu Pro Ala Leu Gly Lys Gly Glu Gly Ala Ile
   625                 630                 635                 640
   Thr Ser Ser Ala Ile Thr Thr Val Ala Ala Glu Ala Pro Val Leu Pro
                   645                 650                 655
   Leu Ser Thr Glu Pro Pro Ala Ala Leu Ala Thr Ser Ala Tyr Thr Cys
               660                 665                 670
   Phe Arg Cys Leu Glu Cys Lys Glu Gln Cys Arg Asp Lys Ala Gly Met
           675                 680                 685
   Ala Ala His Phe Gln Gln Leu Gly Pro Pro Ala Pro Gly Ala Thr Ser
       690                 695                 700
   Asn Val Cys Pro Thr Cys Pro Met Met Leu Pro Asn Arg Cys Ser Phe
   705                 710                 715                 720
   Ser Ala His Gln Arg Met His Lys Asn Arg Pro Pro His Val Cys Pro
                   725                 730                 735
   Glu Cys Gly Gly Asn Phe Leu Gln Ala Asn Phe Gln Thr His Leu Arg
               740                 745                 750
   Glu Ala Cys Leu His Val Ser Arg Arg Val Gly Tyr Arg Cys Pro Ser
           755                 760                 765
   Cys Ser Val Val Phe Gly Gly Val Asn Ser Ile Lys Ser His Ile Gln
       770                 775                 780
   Thr Ser His Cys Glu Val Phe His Lys Cys Pro Ile Cys Pro Met Ala
   785                 790                 795                 800
   Phe Lys Ser Gly Pro Ser Ala His Ala His Leu Tyr Ser Gln His Pro
                   805                 810                 815
   Ser Phe Gln Thr Gln Gln Ala Lys Leu Ile Tyr Lys Cys Ala Met Cys
                   820                 825                 830
   Asp Thr Val Phe Thr His Lys Pro Leu Leu Ser Ser His Phe Asp Gln
           835                 840                 845
   His Leu Leu Pro Gln Arg Val Ser Val Phe Lys Cys Pro Ser Cys Pro
       850                 855                 860
   Leu Leu Phe Ala Gln Lys Arg Thr Met Leu Glu His Leu Lys Asn Thr
   865                 870                 875                 880
   His Gln Ser Gly Arg Leu Glu Glu Thr Ala Gly Lys Gly Ala Gly Gly
                   885                 890                 895
   Ala Leu Leu Thr Pro Lys Thr Glu Pro Glu Glu Leu Ala Val Ser Gln
               900                 905                 910
   Gly Gly Ala Ala Pro Ala Thr Glu Glu Ser Ser Ser Ser Ser Glu Glu
           915                 920                 925
   Glu Glu Val Pro Ser Ser Pro Glu Pro Pro Arg Pro Ala Lys Arg Pro
       930                 935                 940
   Arg Arg Glu Leu Gly Ser Lys Gly Leu Lys Gly Gly Gly Gly Gly Pro
   945                 950                 955                 960
   Gly Gly Trp Thr Cys Gly Leu Cys His Ser Trp Phe Pro Glu Arg Asp
                   965                 970                 975
   Glu Tyr Val Ala His Met Lys Lys Glu His Gly Lys Ser Val Lys Lys
               980                 985                 990
   Phe Pro Cys Arg Leu Cys Glu Arg  Ser Phe Cys Ser Ala  Pro Ser Leu
           995                 1000                 1005
   Arg Arg  His Val Arg Val Asn  His Glu Gly Ile Lys  Arg Val Tyr
       1010                 1015                 1020
   Pro Cys  Arg Tyr Cys Thr Glu  Gly Lys Arg Thr Phe  Ser Ser Arg
       1025                 1030                 1035
   Leu Ile  Leu Glu Lys His Val  Gln Val Arg His Gly  Leu Gln Leu
       1040                 1045                 1050
   Gly Ala  Gln Ser Pro Gly Arg  Gly Thr Thr Leu Ala  Arg Gly Ser
       1055                 1060                 1065
   Ser Ala Arg Ala Gln Gly Pro Gly Arg Lys Arg Arg Gln Ser Ser
   1070 1075 1080
   Asp Ser Cys Ser Glu Glu Pro Asp Ser Thr Thr Pro Pro Ala Lys
   1085 1090 1095
   Ser Pro Arg Gly Gly Pro Gly Ser Gly Gly His Gly Pro Leu Arg
   1100 1105 1110
   Tyr Arg Ser Ser Ser Ser Thr Glu Gln Ser Leu Met Met Gly Leu
   1115 1120 1125
   Arg Val Glu Asp Gly Ala Gln Gln Cys Leu Asp Cys Gly Leu Cys
   1130 1135 1140
   Phe Ala Ser Pro Gly Ser Leu Ser Arg His Arg Phe Ile Ser His
   1145 1150 1155
   Lys Lys Arg Arg Gly Val Gly Lys Ala Ser Ala Leu Gly Leu Gly
   1160 1165 1170
   Asp Gly Glu Glu Glu Ala Pro Pro Ser Arg Ser Asp Pro Asp Gly
   1175 1180 1185
   Gly Asp Ser Pro Leu Pro Ala Ser Gly Gly Pro Leu Thr Cys Lys
   1190 1195 1200
   Val Cys Gly Lys Ser Cys Asp Ser Pro Leu Asn Leu Lys Thr His
   1205 1210 1215
   Phe Arg Thr His Gly Met Ala Phe Ile Arg Ala Arg Gln Gly Ala
   1220 1225 1230
   Val Gly Asp Asn
   1235
<210> 7
   <211> 1237
   <212> PRT
   <213> Homo sapiens
<400> 7
   Met Gly Asp Met Lys Thr Pro Asp Phe Asp Asp Leu Leu Ala Ala Phe
   1 5 10 15
   Asp Ile Pro Asp Ile Asp Ala Asn Glu Ala Ile His Ser Gly Pro Glu
   20 25 30
   Glu Asn Glu Gly Pro Gly Gly Pro Gly Lys Pro Glu Pro Gly Val Gly
           35                  40                  45
   Ser Glu Ser Glu Asp Thr Ala Ala Ala Ser Ala Gly Asp Gly Pro Gly
       50                  55                  60
   Val Pro Ala Gln Ala Ser Asp His Gly Leu Pro Pro Pro Asp Ile Ser
   65                  70                  75                  80
   Val Val Ser Val Ile Val Lys Asn Thr Val Cys Pro Glu Gln Ser Glu
                   85                  90                  95
   Ala Leu Ala Gly Gly Ser Ala Gly Asp Gly Ala Gln Ala Ala Gly Val
               100                 105                 110
   Thr Lys Glu Gly Pro Val Gly Pro His Arg Met Gln Asn Gly Phe Gly
           115                 120                 125
   Ser Pro Glu Pro Ser Leu Pro Gly Thr Pro His Ser Pro Ala Pro Pro
       130                 135                 140
   Ser Gly Gly Thr Trp Lys Glu Lys Gly Met Glu Gly Lys Thr Pro Leu
   145                 150                 155                 160
   Asp Leu Phe Ala His Phe Gly Pro Glu Pro Gly Asp His Ser Asp Pro
                   165                 170                 175
   Leu Pro Pro Ser Ala Pro Ser Pro Thr Arg Glu Gly Ala Leu Thr Pro
               180                 185                 190
   Pro Pro Phe Pro Ser Ser Phe Glu Leu Ala Gln Glu Asn Gly Pro Gly
           195                 200                 205
   Met Gln Pro Pro Val Ser Ser Pro Pro Leu Gly Ala Leu Lys Gln Glu
       210                 215                 220
   Ser Cys Ser Pro His His Pro Gln Val Leu Ala Gln Gln Gly Ser Gly
   225                 230                 235                 240
   Ser Ser Pro Lys Ala Thr Asp Ile Pro Ala Ser Ala Ser Pro Pro Pro
                   245                 250                 255
   Val Ala Gly Val Pro Phe Phe Lys Gln Ser Pro Gly His Gln Ser Pro
               260                 265                 270
   Leu Ala Ser Pro Lys Val Pro Val Cys Gln Pro Leu Lys Glu Glu Asp
           275                 280                 285
   Asp Asp Glu Gly Pro Val Asp Lys Ser Ser Pro Gly Ser Pro Gln Ser
       290                 295                 300
   Pro Ser Ser Gly Ala Glu Ala Ala Asp Glu Asp Ser Asn Asp Ser Pro
   305                 310                 315                 320
   Ala Ser Ser Ser Ser Arg Pro Leu Lys Val Arg Ile Lys Thr Ile Lys
                   325                 330                 335
   Thr Ser Cys Gly Asn Ile Thr Arg Thr Val Thr Gln Val Pro Ser Asp
               340                 345                 350
   Pro Asp Pro Pro Ala Pro Leu Ala Glu Gly Ala Phe Leu Ala Glu Ala
           355                 360                 365
   Ser Leu Leu Lys Leu Ser Pro Ala Thr Pro Thr Ser Glu Gly Pro Lys
       370                 375                 380
   Val Val Ser Val Gln Leu Gly Asp Gly Thr Arg Leu Lys Gly Thr Val
   385                 390                 395                 400
   Leu Pro Val Ala Thr Ile Gln Asn Ala Ser Thr Ala Met Leu Met Ala
                   405                 410                 415
   Ala Ser Val Ala Arg Lys Ala Val Val Leu Pro Gly Gly Thr Ala Thr
               420                 425                 430
   Ser Pro Lys Met Ile Ala Lys Asn Val Leu Gly Leu Val Pro Gln Ala
           435                 440                 445
   Leu Pro Lys Ala Asp Gly Arg Ala Gly Leu Gly Thr Gly Gly Gln Lys
       450                 455                 460
   Val Asn Gly Ala Ser Val Val Met Val Gln Pro Ser Lys Thr Ala Thr
   465                 470                 475                 480
   Gly Pro Ser Thr Gly Gly Gly Thr Val Ile Ser Arg Thr Gln Ser Ser
                   485                 490                 495
   Leu Val Glu Ala Phe Asn Lys Ile Leu Asn Ser Lys Asn Leu Leu Pro
               500                 505                 510
   Ala Tyr Arg Pro Asn Leu Ser Pro Pro Ala Glu Ala Gly Leu Ala Leu
           515                 520                 525
   Pro Pro Thr Gly Tyr Arg Cys Leu Glu Cys Gly Asp Ala Phe Ser Leu
       530                 535                 540
   Glu Lys Ser Leu Ala Arg His Tyr Asp Arg Arg Ser Met Arg Ile Glu
   545 550 555 560
   Val Thr Cys Asn His Cys Ala Arg Arg Leu Val Phe Phe Asn Lys Cys
   565 570 575
   Ser Leu Leu Leu His Ala Arg Glu His Lys Asp Lys Gly Leu Val Met
   580 585 590
   Gln Cys Ser His Leu Val Met Arg Pro Val Ala Leu Asp Gln Met Val
   595 600 605
   Gly Gln Pro Asp Ile Thr Pro Leu Leu Pro Val Ala Val Pro Pro Val
   610 615 620
   Ser Gly Pro Leu Ala Leu Pro Ala Leu Gly Lys Gly Glu Gly Ala Ile
   625 630 635 640
   Thr Ser Ser Ala Ile Thr Thr Val Ala Ala Glu Ala Pro Val Leu Pro
   645 650 655
   Leu Ser Thr Glu Pro Pro Ala Ala Pro Ala Thr Ser Ala Tyr Thr Cys
   660 665 670
   Phe Arg Cys Leu Glu Cys Lys Glu Gln Cys Arg Asp Lys Ala Gly Met
   675 680 685
   Ala Ala His Phe Gln Gln Leu Gly Pro Pro Ala Pro Gly Ala Thr Ser
   690 695 700
   Asn Val Cys Pro Thr Cys Pro Met Met Leu Pro Asn Arg Cys Ser Phe
   705 710 715 720
   Ser Ala His Gln Arg Met His Lys Asn Arg Pro Pro His Val Cys Pro
   725 730 735
   Glu Cys Gly Gly Asn Phe Leu Gln Ala Asn Phe Gln Thr His Leu Arg
   740 745 750
   Glu Ala Cys Leu His Val Ser Arg Arg Val Gly Tyr Arg Cys Pro Ser
   755 760 765
   Cys Ser Val Val Phe Gly Gly Val Asn Ser Ile Lys Ser His Ile Gln
   770 775 780
   Thr Ser His Cys Glu Val Phe His Lys Cys Pro Ile Cys Pro Met Ala
   785                 790                 795                 800
   Phe Lys Ser Gly Pro Ser Ala His Ala His Leu Tyr Ser Gln His Pro
                   805                 810                 815
   Ser Phe Gln Thr Gln Gln Ala Lys Leu Ile Tyr Lys Cys Ala Met Cys
               820                 825                 830
   Asp Thr Val Phe Thr His Lys Pro Leu Leu Ser Ser His Phe Asp Gln
           835                 840                 845
   His Leu Leu Pro Gln Arg Val Ser Val Phe Lys Cys Pro Ser Cys Pro
       850                 855                 860
   Leu Leu Phe Ala Gln Lys Arg Thr Met Leu Glu His Leu Lys Asn Thr
   865                 870                 875                 880
   His Gln Ser Gly Arg Leu Glu Glu Thr Ala Gly Lys Gly Ala Gly Gly
                   885                 890                 895
   Ala Leu Leu Thr Pro Lys Thr Glu Pro Glu Glu Leu Ala Val Ser Gln
               900                 905                 910
   Gly Gly Ala Ala Pro Ala Thr Glu Glu Ser Ser Ser Ser Ser Glu Glu
           915                 920                 925
   Glu Glu Val Pro Ser Ser Pro Glu Pro Pro Arg Pro Ala Lys Arg Pro
       930                 935                 940
   Arg Arg Glu Leu Gly Ser Lys Gly Leu Lys Gly Gly Gly Gly Gly Pro
   945                 950                 955                 960
   Gly Gly Trp Thr Cys Gly Leu Cys His Ser Trp Phe Pro Glu Arg Asp
                   965                 970                 975
   Glu Tyr Val Ala His Met Lys Lys Glu His Gly Lys Ser Val Lys Lys
               980                 985                 990
   Phe Pro Cys Arg Leu Cys Glu Arg  Ser Phe Cys Ser Ala  Pro Ser Leu
           995                 1000                1005
   Arg Arg  His Val Arg Val Asn  His Glu Gly Ile Lys  Arg Val Tyr
       1010                1015                1020
   Pro Cys  Arg Tyr Cys Thr Glu  Gly Lys Arg Thr Phe  Ser Ser Arg
       1025                1030                1035
   Leu Ile  Leu Glu Lys His Val  Gln Val Arg His Gly  Leu Gln Leu
       1040                 1045                 1050
   Gly Ala  Gln Ser Pro Gly Arg  Gly Thr Thr Leu Ala  Arg Gly Ser
       1055                 1060                 1065
   Ser Ala  Arg Ala Gln Gly Pro  Gly Arg Lys Arg Arg  Gln Ser Ser
       1070                 1075                 1080
   Asp Ser  Cys Ser Glu Glu Pro  Asp Ser Thr Thr Pro  Pro Ala Lys
       1085                 1090                 1095
   Ser Pro  Arg Gly Gly Pro Gly  Ser Gly Gly His Gly  Pro Leu Arg
       1100                 1105                 1110
   Tyr Arg  Ser Ser Ser Ser Thr  Glu Gln Ser Leu Met  Met Gly Leu
       1115                 1120                 1125
   Arg Val  Glu Asp Gly Ala Gln  Gln Cys Leu Asp Cys  Gly Leu Cys
       1130                 1135                 1140
   Phe Ala  Ser Pro Gly Ser Leu  Ser Arg His Arg Phe  Ile Ser His
       1145                 1150                 1155
   Lys Lys  Arg Arg Gly Val Gly  Lys Ala Ser Ala Leu  Gly Leu Gly
       1160                 1165                 1170
   Asp Gly  Glu Glu Glu Ala Pro  Pro Ser Arg Ser Asp  Pro Asp Gly
       1175                 1180                 1185
   Gly Asp  Ser Pro Leu Pro Ala  Ser Gly Gly Pro Leu  Thr Cys Lys
       1190                 1195                 1200
   Val Cys  Gly Lys Ser Cys Asp  Ser Pro Leu Asn Leu  Lys Thr His
       1205                 1210                 1215
   Phe Arg  Thr His Gly Met Ala  Phe Ile Arg Ala Arg  Gln Gly Ala
       1220                 1225                 1230
   Val Gly  Asp Asn
       1235
<210> 8
   <211> 1237
   <212> PRT
   <213> Homo sapiens
<400> 8
   Met Gly Asp Met Lys Thr Pro Asp Phe Asp Asp Leu Leu Ala Ala Phe
   1               5                   10                  15
   Asp Ile Pro Asp Ile Asp Ala Asn Glu Ala Ile His Ser Gly Pro Glu
               20                  25                  30
   Glu Asn Glu Gly Pro Gly Gly Pro Gly Lys Pro Glu Pro Gly Val Gly
           35                  40                  45
   Ser Glu Ser Glu Asp Thr Ala Ala Ala Ser Ala Gly Asp Gly Pro Gly
       50                  55                  60
   Val Pro Ala Gln Ala Ser Asp His Gly Leu Pro Pro Pro Asp Ile Ser
   65                  70                  75                  80
   Val Val Ser Val Ile Val Lys Asn Thr Val Cys Pro Glu Gln Ser Glu
                   85                  90                  95
   Ala Leu Ala Gly Gly Ser Ala Gly Asp Gly Ala Gln Ala Ala Gly Val
               100                 105                 110
   Thr Lys Glu Gly Pro Val Gly Pro His Arg Met Gln Asn Gly Phe Gly
           115                 120                 125
   Ser Pro Glu Pro Ser Leu Pro Gly Thr Pro His Ser Pro Ala Pro Pro
       130                 135                 140
   Ser Gly Gly Thr Trp Lys Glu Lys Gly Met Glu Gly Lys Thr Pro Leu
   145                 150                 155                 160
   Asp Leu Phe Ala His Phe Gly Pro Glu Pro Gly Asp His Ser Asp Pro
                   165                 170                 175
   Leu Pro Pro Ser Ala Pro Ser Pro Thr Arg Glu Gly Ala Leu Thr Pro
               180                 185                 190
   Pro Pro Phe Pro Ser Ser Phe Glu Leu Ala Gln Glu Asn Gly Pro Gly
           195                 200                 205
   Met Gln Pro Pro Val Ser Ser Pro Pro Leu Gly Ala Leu Lys Gln Glu
       210                 215                 220
   Ser Cys Ser Pro His His Pro Gln Val Leu Ala Gln Gln Gly Ser Gly
   225                 230                 235                 240
   Ser Ser Pro Lys Ala Thr Asp Ile Pro Ala Ser Ala Ser Pro Pro Pro
                   245                 250                 255
   Val Ala Gly Val Pro Phe Phe Lys Gln Ser Pro Gly His Gln Ser Pro
               260                 265                 270
   Leu Ala Ser Pro Lys Val Pro Val Cys Gln Pro Leu Lys Glu Glu Asp
           275                 280                 285
   Asp Asp Glu Gly Pro Val Asp Lys Ser Ser Pro Gly Ser Pro Gln Ser
       290                 295                 300
   Pro Ser Ser Gly Ala Glu Ala Ala Asp Glu Asp Ser Asn Asp Ser Pro
   305                 310                 315                 320
   Ala Ser Ser Ser Ser Arg Pro Leu Lys Val Arg Ile Lys Thr Ile Lys
                   325                 330                 335
   Thr Ser Cys Gly Asn Ile Thr Arg Thr Val Thr Gln Val Pro Ser Asp
               340                 345                 350
   Pro Asp Pro Pro Ala Pro Leu Ala Glu Gly Ala Phe Leu Ala Glu Ala
           355                 360                 365
   Ser Leu Leu Lys Leu Ser Pro Ala Thr Pro Thr Ser Glu Gly Pro Lys
       370                 375                 380
   Val Val Ser Val Gln Leu Gly Asp Gly Thr Arg Leu Lys Gly Thr Val
   385                 390                 395                 400
   Leu Pro Val Ala Thr Ile Gln Asn Ala Ser Thr Ala Met Leu Met Ala
                   405                 410                 415
   Ala Ser Val Ala Arg Lys Ala Val Val Leu Pro Gly Gly Thr Ala Thr
               420                 425                 430
   Ser Pro Lys Met Ile Ala Lys Asn Val Leu Gly Leu Val Pro Gln Ala
           435                 440                 445
   Leu Pro Lys Ala Asp Gly Arg Ala Gly Leu Gly Thr Gly Gly Gln Lys
       450                 455                 460
   Val Asn Gly Ala Ser Val Val Met Val Gln Pro Ser Lys Thr Ala Thr
   465                 470                 475                 480
   Gly Pro Ser Thr Gly Gly Gly Thr Val Ile Ser Arg Thr Gln Ser Ser
                   485                 490                 495
   Leu Val Glu Ala Phe Asn Lys Ile Leu Asn Ser Lys Asn Leu Leu Pro
               500                 505                 510
   Ala Tyr Arg Pro Asn Leu Ser Pro Pro Ala Glu Ala Gly Leu Ala Leu
               515                 520                 525
   Pro Pro Thr Gly Tyr Arg Cys Leu Glu Cys Gly Asp Ala Phe Ser Leu
           530                 535                 540
   Glu Lys Ser Leu Ala Arg His Tyr Asp Arg Arg Ser Met Arg Ile Glu
   545                 550                 555                 560
   Val Thr Cys Asn His Cys Ala Arg Arg Leu Val Phe Phe Asn Lys Cys
                   565                 570                 575
   Ser Leu Leu Leu His Ala Arg Glu His Lys Asp Lys Gly Leu Val Met
               580                 585                 590
   Gln Cys Ser His Leu Val Met Arg Pro Val Ala Leu Asp Gln Met Val
           595                 600                 605
   Gly Gln Pro Asp Ile Thr Pro Leu Leu Pro Val Ala Val Pro Pro Val
       610                 615                 620
   Ser Gly Pro Leu Ala Leu Pro Ala Leu Gly Lys Gly Glu Gly Ala Ile
   625                 630                 635                 640
   Thr Ser Ser Ala Ile Thr Thr Val Ala Ala Glu Ala Pro Val Leu Pro
                   645                 650                 655
   Leu Ser Thr Glu Pro Pro Ala Ala Pro Ala Thr Ser Ala Tyr Thr Cys
               660                 665                 670
   Phe Arg Cys Leu Glu Cys Lys Glu Gln Cys Arg Asp Lys Ala Gly Met
           675                 680                 685
   Ala Ala His Phe Gln Gln Leu Gly Pro Pro Ala Pro Gly Ala Thr Ser
       690                 695                 700
   Asn Val Cys Pro Thr Cys Pro Met Met Leu Pro Asn Arg Cys Ser Phe
   705                 710                 715                 720
   Ser Ala His Gln Arg Met His Lys Asn Arg Pro Pro His Val Cys Pro
                   725                 730                 735
   Glu Cys Gly Gly Asn Phe Leu Gln Ala Asn Phe Gln Thr His Leu Arg
               740                 745                 750
   Glu Ala Cys Leu His Val Ser Arg Arg Val Gly Tyr Arg Cys Pro Ser
   755 760 765
   Cys Ser Val Val Phe Gly Gly Val Asn Ser Ile Lys Ser His Ile Gln
   770 775 780
   Thr Ser His Cys Glu Val Phe His Lys Cys Pro Ile Cys Pro Met Ala
   785 790 795 800
   Phe Lys Ser Gly Pro Ser Ala His Ala His Leu Tyr Ser Gln His Pro
   805 810 815
   Ser Phe Gln Thr Gln Gln Ala Lys Leu Ile Tyr Lys Cys Ala Met Cys
   820 825 830
   Asp Thr Val Phe Thr His Lys Pro Leu Leu Ser Ser His Phe Asp Gln
   835 840 845
   His Leu Leu Pro Gln Arg Val Ser Val Phe Lys Cys Pro Ser Cys Pro
   850 855 860
   Leu Leu Phe Ala Gln Lys Arg Thr Met Leu Glu His Leu Lys Asn Thr
   865 870 875 880
   His Gln Ser Gly Arg Leu Glu Glu Thr Ala Gly Lys Gly Ala Gly Gly
   885 890 895
   Ala Leu Leu Thr Pro Lys Thr Glu Pro Glu Glu Leu Ala Val Ser Gln
   900 905 910
   Gly Gly Ala Ala Pro Ala Thr Glu Glu Ser Ser Ser Ser Ser Glu Glu
   915 920 925
   Glu Glu Val Pro Ser Ser Pro Glu Pro Pro Arg Pro Ala Lys Arg Pro
   930 935 940
   Arg Arg Glu Leu Gly Ser Lys Gly Leu Lys Gly Gly Gly Gly Gly Pro
   945 950 955 960
   Gly Gly Trp Thr Cys Gly Leu Cys His Ser Trp Phe Pro Glu Arg Asp
   965 970 975
   Glu Tyr Val Ala His Met Lys Lys Glu His Gly Lys Ser Val Lys Lys
   980 985 990
   Phe Pro Cys Arg Leu Cys Glu Arg Ser Phe Cys Ser Ala Pro Ser Leu
   995 1000 1005
   Arg Arg  His Val Arg Val Asn  His Glu Gly Ile Lys  Arg Val Tyr
       1010                 1015                 1020
   Pro Cys  Arg Tyr Cys Thr Glu  Gly Lys Arg Thr Phe  Ser Ser Arg
       1025                 1030                 1035
   Leu Ile  Leu Glu Lys His Val  Gln Val Arg His Gly  Leu Gln Leu
       1040                 1045                 1050
   Gly Ala  Gln Ser Pro Gly Arg  Gly Thr Thr Leu Ala  Arg Gly Ser
       1055                 1060                 1065
   Ser Ala  Arg Ala Gln Gly Pro  Gly Arg Lys Arg Arg  Gln Ser Ser
       1070                 1075                 1080
   Asp Ser  Cys Ser Glu Glu Pro  Asp Ser Thr Thr Pro  Pro Ala Lys
       1085                 1090                 1095
   Ser Pro  Arg Gly Gly Pro Gly  Ser Gly Gly His Gly  Pro Leu Arg
       1100                 1105                 1110
   Tyr Arg  Ser Ser Ser Ser Thr  Glu Gln Ser Leu Met  Met Gly Leu
       1115                 1120                 1125
   Arg Val  Glu Asp Gly Ala Gln  Gln Cys Leu Asp Cys  Gly Leu Cys
       1130                 1135                 1140
   Phe Ala  Ser Pro Gly Ser Leu  Ser Arg His Arg Phe  Ile Ser His
       1145                 1150                 1155
   Lys Lys  Arg Arg Gly Val Gly  Lys Ala Ser Ala Leu  Gly Leu Gly
       1160                 1165                 1170
   Asp Gly  Glu Glu Glu Ala Pro  Pro Ser Arg Ser Asp  Pro Asp Gly
       1175                 1180                 1185
   Gly Asp  Ser Pro Leu Pro Ala  Ser Gly Gly Pro Leu  Thr Cys Lys
       1190                 1195                 1200
   Val Cys  Gly Lys Ser Cys Asp  Ser Pro Leu Asn Leu  Lys Thr His
       1205                 1210                 1215
   Phe Arg  Thr His Gly Met Ala  Phe Ile Arg Ala Arg  Gln Gly Ala
       1220                 1225                 1230
   Val Gly  Asp Asn
       1235
<210> 9
   <211> 20
   <212> DNA
   <213> artificial sequence
<220>
   <223> primer
<400> 9
   cctcctcgtt cctgttttca 20
<210> 10
   <211> 20
   <212> DNA
   <213> artificial sequence
<220>
   <223> primer
<400> 10
   caatggtggg gaagaaacag 20
<210> 11
   <211> 20
   <212> DNA
   <213> artificial sequence
<220>
   <223> primer
<400> 11
   ggacctgttt gctcattttg 20
<210> 12
   <211> 20
   <212> DNA
   <213> artificial sequence
<220>
   <223> primer
<400> 12
   aactgtacgc tcaccacctt 20
<210> 13
   <211> 20
   <212> DNA
   <213> artificial sequence
<220>
   <223> primer
<400> 13
   cgggaatatc acaaggactg 20
<210> 14
   <211> 20
   <212> DNA
   <213> artificial sequence
<220>
   <223> primer
<400> 14
   tataggcagg gagcaggttc 20
<210> 15
   <211> 20
   <212> DNA
   <213> artificial sequence
<220>
   <223> primer
<400> 15
   aatggtgcct cggtggtgat 20
<210> 16
   <211> 20
   <212> DNA
   <213> artificial sequence
<220>
   <223> primer
<400> 16
   gagcatcatg gggcaggttg 20
<210> 17
   <211> 20
   <212> DNA
   <213> artificial sequence
<220>
   <223> primer
<400> 17
   catgctttcg ctgcctggag 20
<210> 18
   <211> 20
   <212> DNA
   <213> artificial sequence
<220>
   <223> primer
<400> 18
   gagggatata gcagggaaga 20
<210> 19
   <211> 20
   <212> DNA
   <213> artificial sequence
<220>
   <223> primer
<400> 19
   cctttctcca ccctgctcat 20
<210> 20
   <211> 20
   <212> DNA
   <213> artificial sequence
<220>
   <223> primer
<400> 20
   acccattccc catccctctg 20
<210> 21
   <211> 20
   <212> DNA
   <213> artificial sequence
<220>
   <223> primer
<400> 21
   ccgtcttgtc ctctgctctt 20
<210> 22
   <211> 20
   <212> DNA
   <213> artificial sequence
<220>
   <223> primer
<400> 22
   aggagagcag gggtagatgt 20
<210> 23
   <211> 20
   <212> DNA
   <213> artificial sequence
<220>
   <223> primer
<400> 23
   gcctggctct gacatctacc 20
<210> 24
   <211> 20
   <212> DNA
   <213> artificial sequence
<220>
   <223> primer
<400> 24
   ccttaccacc acccctactg 20
<210> 25
   <211> 20
   <212> DNA
   <213> artificial sequence
<220>
   <223> primer
<400> 25
   gctgtgctag ggctttgagt 20
<210> 26
   <211> 20
   <212> DNA
   <213> artificial sequence
<220>
   <223> primer
<400> 26
   aggcagcagt agggaaaacc 20
<210> 27
   <211> 19
   <212> DNA
   <213> artificial sequence
<220>
   <223> primer
<400> 27
   aggagtcgtc ttcatcttca 20
<210> 28
   <211> 20
   <212> DNA
   <213> artificial sequence
<220>
   <223> primer
<400> 28
   ttcactgact tgccatgctc 20
<210> 29
   <211> 20
   <212> DNA
   <213> artificial sequence
<220>
   <223> primer
<400> 29
   tggcgtcgtg attagtgatg 20
<210> 30
   <211> 20
   <212> DNA
   <213> artificial sequence
<220>
   <223> primer
<400> 30
   gcacacagag ggctacaatg 20

## Description

The present invention falls within the field of diagnostic markers.

More particularly, the present invention relates to a new diagnostic marker for Paget's disease of bone and bone tumors associated therewith, such as for example giant cell tumors (GCT) and osteosarcoma.

Paget's disease of bone is a metabolic disorder characterized by focal abnormalities in bone remodeling, which typically causes deformity and increased bone size in one or more areas of the skeleton (monostotic form or polyostotic form). In some cases, patients affected by Paget's disease of bone - and particularly those affected by its polyostotic form - incur into neoplastic degeneration of the affected bones (GCT), caused by uncontrolled proliferation of the spindle-shaped stromal cells.

The primary cell abnormality in Paget's disease of bone relates to osteoclasts, which show many functional and morphological abnormalities.

Paget's disease of bone is also characterized by specific modifications of the biochemical distribution of non-collagenous proteins in the bone matrix immediately adjacent to Haversian canals and by bone neoangiogenesis. Some of these modifications are very similar to those found in bone metastases in cancer patients and in patients affected by multiple myeloma.

Epidemiological data point out that Paget's disease of bone may be considered as a disease that predisposes to the appearance of primary bone neoplasms in subjects over 40 years old. The connection between Paget's disease of bone and an increased risk of malignant transformation of the affected bones has been described for the first time by Sir James Paget in his first paper on osteitis deformans in 1876. Since then, several literature papers reported a higher incidence of osteosarcomas and GCT in patients affected by Paget's disease of bone, and neoplastic degeneration of bones affected by Paget's disease of bone was estimated to occur in 1% of patients, with an increase in the risk as high as 30 times over that of the general population (Roodman 2001, Gebhart 1998).

The European patent application EP 1559781A discloses a method of detecting Paget's disease of bone by associating said disease with the mutation of a gene coding for chondroitin synthase.

In 2002, a few mutations were identified which affect the ubiquitination domain of the protein encoded by the SQSTM1 gene which are detected in 10% and 40% of cases of sporadic and familial Paget's disease of bone, respectively (Laurin et al 2002, Hocking et al 2002).

More recently, other genes (CSF1, OPTN, TNFRSF11A, PML, RIN3, NUP205, TM7SF4) have been associated with the Paget's disease of bone in patients without the mutation of SQSTM1 (Albagha et al 2010, Gianfrancesco et al 2012, Albagha et al 2011).

A familial clustering was also demonstrated in a few Paget's pedigrees affected by GCT and less frequently by osteosarcoma, particularly in patients of Italian origin (Rendina et al 2004, Gianfrancesco et al 2013, Wu et al 1991).

The International patent application WO2011/128646 describes a method of diagnosis for Paget's disease of bone, which comprises testing a sample from a subject for sequence changes at or within one or more chromosome loci selected from the group consisting of 10p13, 1p13.3, 18q21, 8q22.3, 7q33, 14q32.12, 15q24.1, 6p22.3 and Xq24.

However, the currently known diagnostic markers for Paget's disease of bone have the disadvantage of not being suitable for diagnosing the clinically serious forms of the disease, such as for example those with neoplastic degeneration of the affected bones. These families do not have mutations in the SQSTM1 gene.

Therefore, there is a need for a new diagnostic marker for Paget's disease of bone which overcomes the drawbacks of the prior art, which particularly allows for diagnosing and predicting the risk of developing bone tumors associated with Paget's disease of bone.

Such a need has now been met by the present inventors who identified a new diagnostic marker for Paget's disease of bone and/or bone tumors associated therewith, particularly osteosarcomas and giant cell tumors, which does not show the above-mentioned drawbacks.

Such a new marker is the ZNF687 gene, which encodes for the zinc finger protein 687, a component of the transcriptional coregulator Z3 complex.

The International patent application WO 02/068579 discloses the entire ZNF687 nucleic acid sequence containing two mutations, 276C>T and 1906G>A, and its use as an array probe.

By whole-exome sequencing and by examining a family including 14 members affected by Paget's disease of bone, four of which had developed GCT at Paget's sites, the present inventors detected a missense mutation in the ZNF687 gene (c.2810C>G, P937R). The sequencing of 7 non-related patients affected by GCT as a complication of Paget's disease of bone has allowed for identifying the same mutation in all of the patients, which suggests a founder effect. The same mutation and also a second missense mutation in the same gene (c.725G>T, p.Ser242Ile) were observed in patients with a serious form of Paget's disease of bone. Furthermore, in a non-pagetic patient, affected by GCT and with a family history of Paget's disease of bone (one parent affected) a third mutation was identified (c. 1994 C>T, P665L) in the ZNF687 gene.

The ZNF687 gene, which encodes for a member of the transcriptional regulation Z3 complex, is highly expressed during osteoblastogenesis and osteoclastogenesis, and is dramatically upregulated in tumor tissue derived from patients affected by giant cell tumor/Paget's disease of bone. ZNF687 knockdown results in a marked decrease in transcription of 2-IIIc receptor of FGF (FGFR2IIIc), revealing a tight correlation between these two genes. Moreover, by immunohistochemical analysis of patients affected by giant cell tumor/Paget's disease of bone, the inventors observed a strong FGFR2IIIc staining in multinucleated giant cells and stromal cells, which supports the role of FGFR2IIIc in the pathogenesis of these tumors.

These and other experimental findings, which will be described in detail in the experimental section that follows, allowed the inventors to conclude that the detection of mutations in the nucleotide sequence of the ZNF687 gene represents a useful diagnostic tool for Paget's disease of bone and bone tumors associated therewith, particularly giant cell tumors and osteosarcomas, as well as a useful prognostic tool for establishing the predisposition of a subject affected by Paget's disease of bone of developing a bone tumor associated therewith, particularly giant cell tumor or osteosarcoma.

The wild-type nucleotide sequence of the ZNF687 gene is illustrated in SEQ ID NO:1.

One aspect of the present invention is thus an *in vitro* or *ex vivo* method for diagnosing Paget's disease of bone or bone tumor, which comprises the step of determining, in a nucleic acid sample of a subject suspected of being affected by Paget's disease of bone or bone tumor, the presence of at least one mutation in the wild-type nucleotide sequence of the ZNF687 gene as represented by SEQ ID NO:1, the presence of said at least one mutation being indicative of Paget's disease of bone or bone tumor.

A further aspect of the present invention is an *in vitro* or *ex vivo* method for determining the predisposition of a subject affected by Paget's disease of bone to develop a bone tumor, which comprises the step of determining, in a nucleic acid sample of a subject affected by Paget's disease of bone, the presence of at least one mutation in the wild-type nucleotide sequence of the ZNF687 gene as represented by SEQ ID NO:1, the presence of said at least one mutation being indicative of a predisposition to develop a bone tumor.

In a preferred embodiment of both of the methods mentioned above, the bone tumor is a giant cell tumor or an osteosarcoma.

In another preferred embodiment, the mutation is a missense mutation.

Examples of missense mutations suitable for use in the methods of the invention are the nucleotide mutation C>G at nucleotide position 2810 of SEQ ID NO:4, the nucleotide mutation G>T at nucleotide position 725 of SEQ ID NO:2 and the nucleotide mutation C>T at nucleotide position 1994 of SEQ ID NO:3. Further mutations particularly suitable to be detected in the methods of the invention are mutations located in the coding region of the gene.

The mutations can be detected by any known method, for example by nucleic acid amplification reaction and detection of the amplification product or by sequencing the whole nucleotide sequence of the ZNF687 gene or a portion thereof and comparing it with the wild-type sequence of ZNF687, used as a known control sequence.

These are per se known methodologies and the application thereof to the methods of the invention is well within the ability of the person of ordinary skill in the art.

Another aspect of the invention is an isolated nucleic acid, preferably a genomic DNA, mRNA or cDNA, as defined in claim 1.

The isolated nucleic acid of the invention comprises the coding region of the ZNF687 gene and the mutation is located in said coding region. As previously indicated with reference to the methods of the invention, the mutation is a missense mutation, particularly the nucleotide mutation C>G at nucleotide position 2810 of SEQ ID NO:4 or the nucleotide mutation G>T at nucleotide position 725 of SEQ ID NO:2 or the nucleotide mutation C>T at nucleotide position 1994 of SEQ ID NO:3. Therefore, preferred isolated nucleic acids according to the invention are nucleic acids with the sequence SEQ ID NO:2 or SEQ ID NO:3 or SEQ ID NO:4.

Such nucleic acids are suitable for use in the manufacture of the corresponding mutated protein, by using recombinant DNA methodologies which are per se known and within the ability of the person of ordinary skill in the art.

A further aspect of the present invention is thus an expression vector including a nucleic acid as previously defined, as well as a host cell transformed with the said vector.

The host cell of the invention is capable of expressing the mutated ZNF687 protein, for instance the protein with the sequence SEQ ID NO:5 or SEQ ID NO:6 or SEQ ID NO:7.

Such mutated ZNF687 protein is in turn useful for the manufacture of specific antibodies, for example monoclonal or polyclonal, chimeric or humanized antibodies, which are manufactured by conventional methods, the accomplishment of which is within the ability of the person of ordinary skill in the art. The said antibodies are useful as assay reagents for carrying out immunodiagnostic methods within the scope of the present invention, such as for example ELISA immunoassay methods.

Therefore, another aspect of the present invention is an *in vitro* or *ex vivo* method for diagnosing Paget's disease of bone or bone tumor, which comprises the step of determining, in a biological sample from a subject suspected of being affected by Paget's disease of bone or bone tumor, the presence of at least one ZNF687 protein which is mutated compared to the wild-type ZNF687 protein sequence as represented by SEQ ID NO:8, the presence of said at least one mutated protein being indicative of Paget's disease of bone or bone tumor.

Still another aspect of the present invention is an *in vitro* or *ex vivo* method for determining the predisposition of a subject affected by Paget's disease of bone to develop a bone tumor, which comprises the step of determining, in a biological sample from a subject affected by Paget's disease of bone, the presence of at least one ZNF687 protein which is mutated compared to the wild-type ZNF687 protein sequence as represented by SEQ ID NO:8, the presence of said at least one mutated protein being indicative of a predisposition to develop a bone tumor.

In both of the above-mentioned methods, the mutated ZNF687 protein compared to the wild-type ZNF687 protein sequence represented by SEQ ID NO:8 is preferably selected from SEQ ID NO:5, SEQ ID NO:6 and SEQ ID NO:7.

The part that follows, related to Examples and Experimental section, is provided only by way of illustration and not as a limitation of the scope of the invention as defined in the appended claims.

### Examples

### 1. Experimental section

### METHODS

### Whole-exome sequencing strategy.

The DNA from the four subjects belonging to the pedigree with GCT/PDB was used for exome sequencing by using the NimbleGen SeqCap EZ Exome TM capture kit (Roche) and the resulting fragments were sequenced by Illumina Genome Analyzer IIx (Illumina, San Diego, CA, USA) with sequences of 90 bp in length. 58.458.230 sequences for sample V-4, 55.895.176 sequences for sample V-18, 45.223.865 sequences for sample V-11 and finally 50.109.914 sequences for sample V-7 were obtained. These sequences were aligned with human genomic sequence GRCh37/hg19 with the software MAQ7 and NextGENe v2.00. A coverage of over 45X for each sample, the sequencing of all the exons and the 5' and 3' untranslated regions were obtained. The analysis for identification of variations was carried out with the DNA bioinformatics platform Nexus. The variants obtained were filtered with those present in the SNP database (dbSNP131). Assuming the synonymous variants had less chances of being pathogenetic, the inventors only focused on non-synonymous variants (NS), mutations at splice donor and acceptor sites (SS), and small coding insertions or deletions (I).

### Mutational analysis.

The analysis for validation and segregation of the selected variants was performed by Sanger sequencing. PCR amplification and direct sequencing protocols have been described previously (Gianfrancesco et al. 2012). Oligonucleotides used for amplifying and sequencing the ZNF687 gene are:
exon 2A:
   5'-CCTCCTCGTTCCTGTTTTCA-3' (SEQ ID NO:9)
   5'-CAATGGTGGGGAAGAAACAG-3' (SEQ ID NO:10)
exon 2B:
   5'-GGACCTGTTTGCTCATTTTG-3' (SEQ ID NO:11)
   5'-AACTGTACGCTCACCACCTT-3' (SEQ ID NO:12)
exon 2C:
   5'-CGGGAATATCACAAGGACTG-3' (SEQ ID NO:13)
   5'-TATAGGCAGGGAGCAGGTTC-3' (SEQ ID NO:14)
exon 2D:
   5'-AATGGTGCCTCGGTGGTGAT-3' (SEQ ID NO:15)
   5'-GAGCATCATGGGGCAGGTTG-3' (SEQ ID NO:16)
exon 3:
   5'-CATGCTTTCGCTGCCTGGAG-3' (SEQ ID NO:17)
   5'-GAGGGATATAGCAGGGAAGA-3' (SEQ ID NO:18)
exons 4-5:
   5'-CCTTTCTCCACCCTGCTCAT-3' (SEQ ID NO:19)
   5'-ACCCATTCCCCATCCCTCTG-3' (SEQ ID NO:20)
exon 6:
   5'-CCGTCTTGTCCTCTGCTCTT-3' (SEQ ID NO:21)
   5'-AGGAGAGCAGGGGTAGATGT-3' (SEQ ID NO:22)
exons 7-8:
   5'-GCCTGGCTCTGACATCTACC-3' (SEQ ID NO:23)
   5'-CCTTACCACCACCCCTACTG-3' (SEQ ID NO:24)
exon 9:
   5'-GCTGTGCTAGGGCTTTGAGT-3' (SEQ ID NO:25)
   5'-AGGCAGCAGTAGGGAAAACC-3' (SEQ ID NO:26)

### Expression analysis

In order to analyze the expression pattern of the ZNF687 gene, a real-time PCR was carried out on total RNA derived from human adult tissues purchased from Stratagene with the Cycler DNA Engine Opticon 2 system (Biorad). One microgram of total RNA was reverse transcribed with the RevertAid RT kit (Thermo Scientific). The expressions were normalized to the HPRT gene to account for differences in the starting material and in the cDNA reaction efficiency. An electrophoresis on agarose gel was performed to further confirm the specificity of the PCR products. The oligonucleotides of the ZNF687 transcript (5'-AGGAGTCGTCTTCATCTTCA-3' (SEQ ID NO:27) and 5'-TTCACTGACTTGCCATGCTC-3' (SEQ ID NO:28) were used to generate a product of 214 bp. The HPRT oligonucleotides (5'-TGGCGTCGTGATTAGTGATG-3' (SEQ ID NO:29) and 5'-GCACACAGAGGGCTACAATG-3' (SEQ ID NO:30) were used to generate a product of 185 bp.

### Cultures and primary cell lines.

Cell lines MG63, RAW264.7, U2OS and HEK293 were plated on 60-mm plates using DMEM culture medium (Gibco) supplemented with 10% fetal bovine serum, 2mM L-glutamine, 100 U/mL penicillin and 100 mg/mL streptomycin (Gibco) until they reached a 70-80% confluence.

### siRNA transfection

1.5 x 10⁵ cells were plated on 60-mm plates. 24 hours later, MG63 cells were transfected with a mixture of small interfering RNAs ZNF687 10 nM (siRNAs) Duplex Trilencer-27 (Origene) and 26.5 uL of IBAFECT reagent (Iba Solutions For Life Sciences) in OPTIMEM (1X)+ GlutaMax-I reduced serum media (Gibco, Life Technologies). Each transfection was performed in triplicate and repeated four times. 48 and 72 hours post-transfection, the cells were blocked to isolate RNA and proteins.

### Protein extraction and Western Blot analysis for ZNF687 and FGFR2IIIc

After 48 and 72 hours of incubation, MG63, RAW264.7 and U2OS cells were lysed with lysis buffer (5 ml 100% glycerol, 50 µl tween20, 500 µl Triton 100, 3.5 ml 5M NaCl, 2.5 ml 1M Tris pH 7.9, 250 µl 1M MgCl₂, all in a final volume of 50 ml). The lysed cells were centrifuged at 14000 g for 5 minutes. Proteins were denatured by boiling in sample buffer, separated on 8% SDS-PAGE, transferred onto an Immun-Blot PVDF membrane for protein blotting (BIO-RAD) and blocked for 1 hour in 5% dried skimmed milk in TBS-T (10 mM Tris-HCl pH 7.5, 100 mM NaCl, 0.1% (v/v) Tween-20). Antibodies used for protein recognition are: anti-ZNF687 polyclonal rabbit antibody ab105544 (abcam 1:500), anti-Bek polyclonal rabbit antibody (c-17): sc-122 (Santacruz 1:100) and anti-B actin polyclonal mouse antibody (c4) sc-47778 (Santacruz 1:1000). Goat anti-rabbit antibodies conjugated with peroxidase (1:1.000 for ZNF687 protein and 1:2000 for FGFR2 protein and anti-mouse (diluted 1:15000 in TBST) (Biorad) were used as secondary antibodies. The Western Blot assay was quantified with the Image J program.

### Osteoclast formation assay

Peripheral blood mononuclear cells were separated by Ficoll density gradient centrifugation and the cells in suspension were plated in 24-well plates (3 x 10⁵). Osteoclasts were generated starting from cells cultured for 14-21 days in alpha-minimal essential medium (a-MEM) (SIGMA, Life Science) containing 10% fetal bovine serum (FBS), 2 mM L-glutamine, 100 U/ml penicillin and 100 mg/ml streptomycin (Gibco) as well as 25 ng/mL macrophage colony stimulating factor (M-CSF) (Peprotech, Rocky Hill, NJ, USA) and 30 ng/mL soluble human recombinant sRANK ligand (RANKL) (Peprotech, Rocky Hill, NJ, USA).

### RESULTS

### Identification of the gene responsible for Giant Cell Tumor associated with Paget's disease.

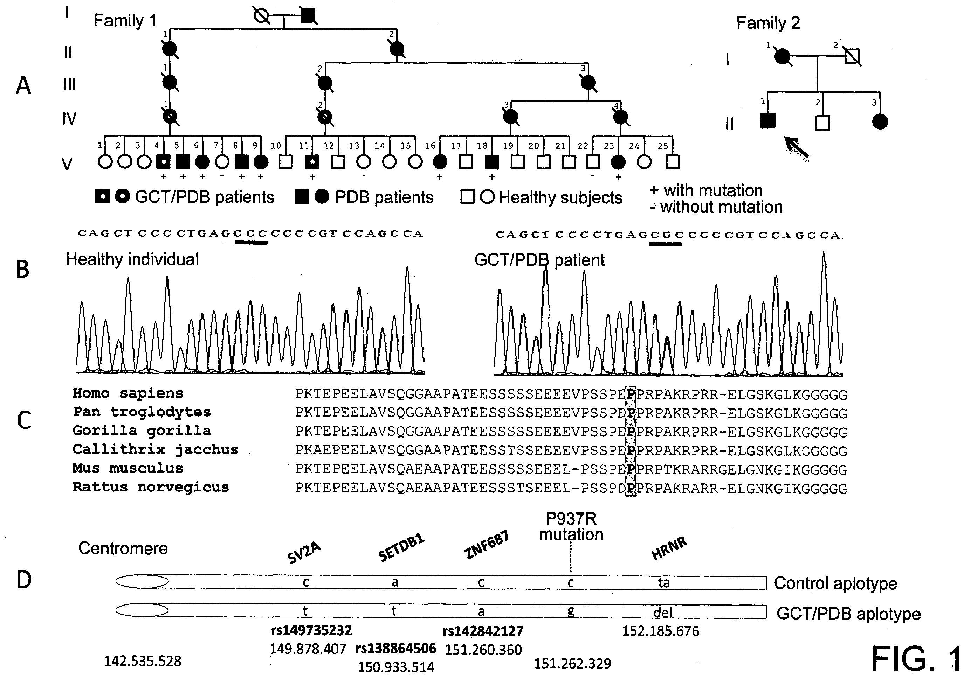

Recently, the inventors described a family with 14 individuals affected by Paget's disease, 4 of which with giant cell tumor (Gianfrancesco et al 2013). A whole-exome sequencing was carried out on the DNA of three patients affected by PDB (V-4, V-18, V-11), two of which with giant cell tumor, and of one healthy subject (V-7) (Figure 1A). By adopting a selection scheme, the variants recorded in the SNP database (dbSNP131), and then those present in 4 exomes available at the inventors' laboratory and derived from 4 subjects affected by diseases that are not related with the tested one, were excluded. After such a filtering, the data from the three affected individuals were compared, with the exception of variants present in the healthy subject, identifying 57 variations in 45 candidate genes. Twenty-one of these variants were confirmed by Sanger direct sequencing in the three patients, yet only one of these showed a complete segregation with the disease phenotype in the family: c.2810C>G that determines the amino acid substitution P937R, in exon 6 of the ZNF687 gene located on chromosome 1 (1q21.3) in one of the linkage regions described in the previous work (Gianfrancesco et al 2013). The mutation is heterozygous in patients and is absent in non-affected individuals (Figure 1B). Such a mutation has not been detected in a cohort of 564 control individuals from the same geographical area and the evolutionary analysis has shown that proline 937 is conserved in all mammals, suggesting its important biological role (Figure 1C). To validate the results, the inventors analyzed the ZNF687 gene in 7 patients independently affected by GCT/PDB, 4 of which were from the same localized area of South Italy and three were Italian patients or American patients with Italian ancestors (Rendina et al 2006, Gennari et al 2010, Michou et al 2011). Interestingly, the same mutation c.2810C>G was detected in all GCT/PDB patients, suggesting they share a common founder mutation. To validate this hypothesis, the segregation of rare variants was assessed in genes surrounding mutation c.2810C>G, detecting heterozygous variations for SNPs rs149735232 (SV2A), rs138864506 (SETDB1), rs142842127 (ZNF687), of TA in position 152.185.676 of the HRNR gene. This aplotype was not found in any of the 559 tested controls of the same geographical area, suggesting that the mutation is located on a common aplotype which identifies all GCT/PDB patients (Figure 1D).

### Molecular analysis of the ZNF687 gene in patients affected by Paget's disease without neoplastic degeneration.

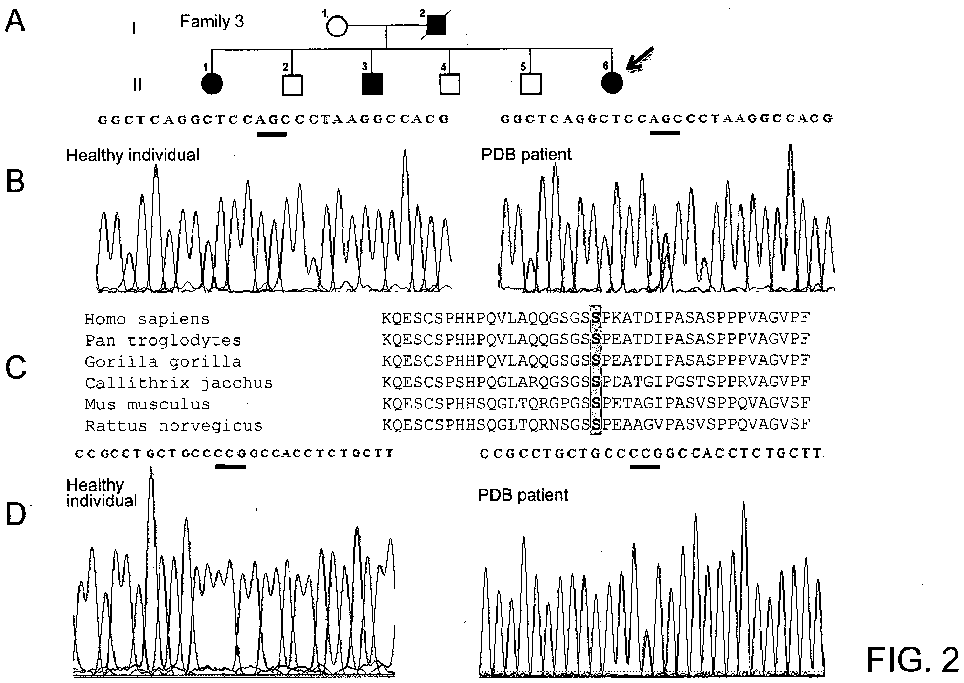

To verify the role of the ZNF687 gene in Paget's patients without neoplastic degeneration, the whole coding region of the gene, consisting of 9 exons, was analyzed in 22 of 37 Paget's subjects who had tested negative for mutations in the SQSTM1 gene. Each of the tested subjects had at least one other family member with the disease, confirming the autosomal dominant inheritance of the disease in these families. This analysis allowed for detecting in the tested subject of family 2 (Figure 1A) the same mutation c.2810C>G (P937R) indicated above and a new mutation, c.725G>T (S242I) in exon 2 of the tested subject of family 3 (Figure 2A). The p.P937R mutation segregates with the disease phenotype in family 2, it being present in patient II-3, but not in the healthy sibling II-2. Also the p.S242I mutation segregates with the disease phenotype, it being present in the two affected siblings (II-1, II-3, II-6) while it has not been detected in the non-affected members (II-2, II-4, II-5) (Figures 2A-2B). This mutation, which determines the substitution of an isoleucine for a serine in position 242 (p.S242I), is not reported in the database "1000 Genomes". Serine in position 242 is highly conserved in mammals (Figure 2C) and the substitution thereof is predicted as detrimental by the prediction programs SIFT, PolyPhen and Align GVGD. In order to verify the presence of mutations in the ZNF687 gene also in the GCT not related to the Paget's disease of bone (conventional GCT), the genetic analysis was performed in 44 patients affected by such disease, 38 of which had somatic mutations in the H3F3A gene. This analysis resulted in the identification of another mutation (c. 1994 C>T, P665L) in exon 2 of the ZNF687 gene (Figure 2D) in a patient whose anamnesis had not shown a family history of Paget's disease of bone.

The c.2810C>G mutation (p.937R) was assessed by the Taqman assay in two cohorts of Paget's patients of different ethnic groups. It has been identified in 2 Italian patients out of 628 and in 2 patients out of 339 of the multi-ethnic cohort (Michou et al 2011). Moreover, this mutation was not detected in 564 control individuals of Italian origin and in 269 control individuals of American origin. All the patients bearing the mutation in the ZNF687 gene showed a very serious form of the disease, predicting that these patients are predisposed to development of neoplastic complications and that the pharmacological treatment prevents the onset thereof. In fact, all GCT/PDB cases show a decreased response to treatment with bisphosphonates and an active persistency of the disease, with ALP levels well over the normal value range. Together, these data suggest that the persistency of an active Paget's disease may increase the risk of GCT complications, at least in genetically predisposed subjects.

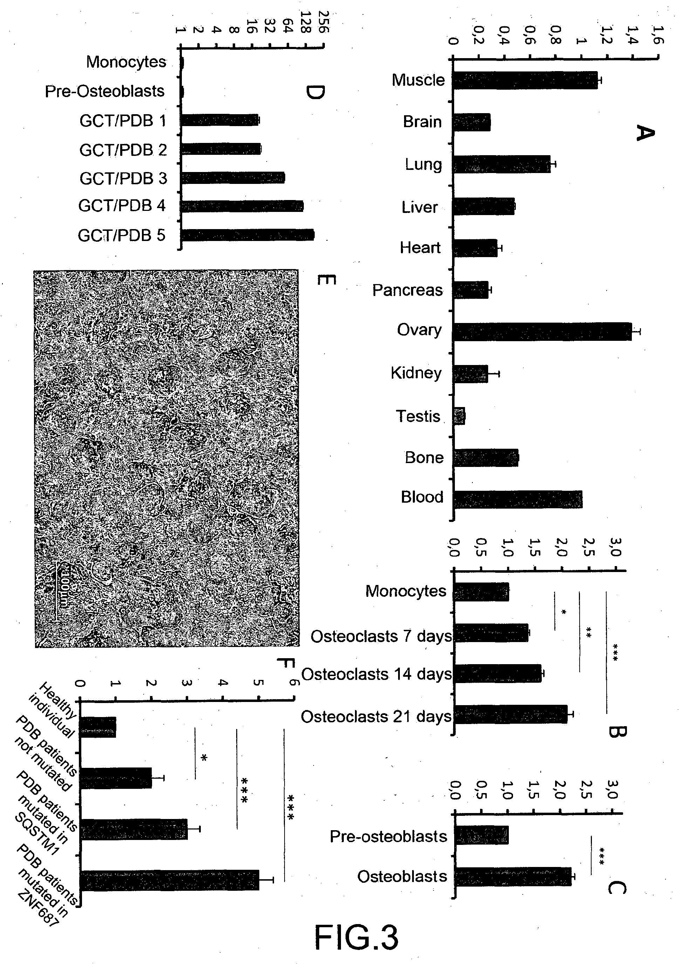

Expression of the ZNF687 gene increases during the differentiation of osteoclasts and osteoblasts. The expression profile of the ZNF687 gene was analyzed by using a panel of RNAs extracted from human adult tissues through a real-time PCR assay, revealing its expression in all the examined tissues (Figure 3A). The expression of the gene was also analyzed during osteoclastogenesis induced on peripheral blood monocytes through treatment with MCSF and RANKL. Expression of ZNF687 increases significantly during the differentiation process (Figure 3B). This result was also confirmed during the differentiation process of osteoclasts induced in murine cells. Expression of ZNF687 mRNA also increases in differentiated osteoblasts compared to their undifferentiated precursors (Figure 3C). These data suggest a role for ZNF687 during differentiation of osteoclasts and osteoblasts, showing its involvement in the regulation of genes implicated in these two processes. The giant cell tumor that develops on pagetic bone is the consequence of a modification of both processes. Indeed, in RNA extracted from the giant cell tumor of pagetic patients, which includes the giant cells derived from osteoclasts and the stromal cells of osteoblastic origin, the inventors found a significant increase in ZNF687 expression (Figure 3D). Finally, through immunohistochemical techniques, by using specific antibodies against the ZNF687 protein, a very intense staining was observed in the nucleus of the tumor giant cells in GCT/PDB patients compared to PDB patients (Figure 3E).

By Real Time PCR, the expression level of ZNF687 in the peripheral blood of PDB patients without mutations in the SQSTM1, PDB patients with mutations in the SQSTM1 gene and PDB patients with mutations in the ZNF687 gene, was also evaluated. Interestingly, the ZNF687 expression levels are significantly higher in PDB patients with or without a SQSTM1 mutation compared to healthy control subjects (Figure 3F). Furthermore, a greater increase (5-folds) was observed in PDB patients with ZNF687 mutations. These results suggest that ZNF687 is involved in the ethiopathogenesis of Paget's disease of bone, independently of the patients' genetic background. Its expression profile considerably correlates with the clinical seriousness of the disease.

### ZNF687 regulates the expression of the receptor FGFR2.

Recently, a transcriptional coregulating complex designated as Z3, formed by proteins ZMYND8, ZNF687 and ZNF592, was identified and its interaction with the ERα transcription factor was defined (Malovannaya et al 2011). Bioinformatic analyses allowed for hypothesizing that the expression of the FGFR2 gene, which has in its genomic region a binding site for the Z3 complex (recognized in Chip-Seq experiments by ZMYND8 antibody), may be regulated thereby. ZNF687 was silenced by use of a specific siRNA in cell lines MG63 (osteoblast line) and RAW264.7 (osteoclast line). 72 hours later, a decrease in FGFR2IIIc was observed both at the mRNA and the protein level in both the cell lines. These results point out that FGFR2IIIc is regulated by the Z3 complex. As for the ZNF687 gene, an increase was also observed in the expression of FGFR2 during cell differentiation of osteoclasts and osteoblasts. Finally, in sections of tumor tissue of GCT/PDB patients, an increase in the expression of the FGFR2 protein, apart from in mesenchymal stromal cells, was also confirmed in multinucleated giant cells, supporting its role in the pathogenesis of this tumor.

### 2. Diagnostic kit according to the invention - assay by nucleotide sequencing

An example of a diagnostic kit for identifying, by nucleotide sequencing, the causative mutation c.2810C>G (P937R) in exon 6 of the ZNF687 gene comprises:
- a pair of oligonucleotides (sense 5'-CCGTCTTGTCCTCTGCTCTT-3' (SEQ ID NO:21), anti-sense 5'-AGGAGAGCAGGGGTAGATGT-3' (SEQ ID NO:22) specific for the exponential amplification reaction of a fragment of the human ZNF687 gene where the causative mutation is located;
- a single oligonucleotide (sense 5'-CCGTCTTGTCCTCTGCTCTT-3' (SEQ ID NO:21) specific for the sequencing of the sense strand of the amplified fragment;
- a single oligonucleotide (anti-sense 5'-AGGAGAGCAGGGGTAGATGT-3' (SEQ ID NO:22) specific for the sequencing of the anti-sense strand of the amplified fragment;
- a control DNA from a healthy individual not bearing the test mutation;
- a 5X master mix (5 times more concentrated than the working reaction) comprising: 1mM of a mixture of the four deoxynucleotides (dATP, dCTP, dTTP, dGTP), 5 x reaction buffer (100 mM Tris, 50mM Hepes, 12.5 mM Magnesium Sulfate, 50 mM Potassium Chloride and 50 mM Ammonium Sulfate) and 5 Units of Taq polymerase.

### Amplification protocol.

In a final volume of 25 microlitres, 100 ng of genomic DNA are combined with 0, 5 microM of each oligonucleotide, 5 microlitres of 5X Master mix and H₂O.

The exponential amplification reaction comprises a first cycle of 3 minutes at 94°C for the denaturation of the genomic DNA, followed by 40 cycles that comprise: 30 seconds at 94°C, 30 seconds at 62°C, 45 seconds at 72°C; a final cycle at 72°C for 10 minutes. The PCR product can be visualized on a 1.5% agarose gel.

### Sequencing

The amplification product is purified with the enzyme exosap and sequenced in a final volume of 20 microlitres with 2 micromolar oligonucleotide F or R and the Dye Terminator Ready Reaction Kit (Applied Biosystem). The reactions are carried out for 25 cycles that comprise: one step at 95°C for 10 sec, 55°C for 5 sec and 60°C for 2 min. The sequencing reactions are purified and separated on ABI prism 3700 Genetic Analyser (Applied Biosystems).

### 3. Diagnostic kit according to the invention - assay by the TaqMan method

The diagnostic kit for identifying the causative mutation c.2810C>G (P937R) in exon 6 of the ZNF687 gene comprises:
- a pair of oligonucleotides specific for the exponential amplification reaction of a fragment of the human ZNF687 gene where the causative mutation is located;
- a forward (F) oligonucleotide probe specific for the mutated base associated with a reporter fluorochrome at the 5' end.

### Taqman Genotyping Assay

The diagnostic kit for identifying by the TaqMan method the causative mutation c.2810C>G in exon 6 of the ZNF687 gene comprises:
Two TaqMan® MGB probes, of which:
- one probe labeled with the fluorochrome VIC® for recognition of the Allele 1 sequence (wild type);
- one probe labeled with the fluorochrome FAM™ for recognition of Allele 2 (mutated);
- two sequence-specific sense and anti-sense oligonucleotides for amplifying the sequence of interest;
- Master Mix TaqMan® Universal PCR UP (Ultra Pure) containing: AmpliTaq Gold® DNA polymerase (IX), the fluorochrome ROX™ as a passive internal reference for assuring a better analysis on the real-time PCR tools, a commercially available reaction buffer and a mixture of the four deoxynucleotides (dATP, dCTP, dTTP, dGTP).

### Amplification protocol

In a final volume of 25 µL, 1-20 ng of genomic DNA are combined with 12.5 µL of TaqMan Universal PCR Master Mix (2X), 1.25 µL of the mixture of oligonucleotides and sequence-specific probes (20X) and sterile water to reach the final volume.

### Exponential Amplification Reaction

A first cycle of 10 minutes at 95°C is carried out for activating AmpliTaq Gold® DNA Taq polymerase, followed by 40 cycles that comprise: 15 seconds at 92°C for the denaturation of the genomic DNA and 1 minute at 60°C for assuring the annealing of the oligonucleotides and the sequence-specific probes and the subsequent extension of the fragment of interest. By using the thermocycler Applied Biosystems 7900HT Fast Real-Time PCR System, amplification curves are generated which may then be analyzed through the SDS Software v2.2.2 or subsequently for the 7900HT Fast System.

### References

- Roodman GD. Studies in Paget's disease and their relevance to oncology. Semin Oncol. 2001;15-21.
- Gebhart M, Vandeweyer E, Nemec E. Paget's disease of bone complicated by Giant Cell Tumor. Clin Orthop Relat Res. 1998;352:187-93.
- Laurin N, Brown JP, Morissette J, Raymond V. Recurrent mutation of the gene encoding sequestosome 1 (SQSTM1/p62) in Paget disease of bone. Am J Hum Genet 2002;70:1582-88.
- Hocking LJ, Lucas GJ, Daroszewska A, et al. Domain specific mutations in Sequestosome 1 (SQSTM1) cause familial and sporadic Paget's disease. Hum Mol Genet 2002; 11 :2735-39.
- Albagha OM, Visconti MR, Alonso N, Langston AL, Cundy T, Dargie R, Dunlop MG, Fraser WD, Hooper MJ, Isaia G, Nicholson GC, del Pino Montes J, Gonzalez-Sarmiento R, di Stefano M, Tenesa A, Walsh JP, Ralston SH. Genome-wide association study identifies variants at CSF1, OPTN and TNFRSF11A as genetic risk factors for Paget's disease of bone. Nat Genet 2010;42:520-4.
- Albagha OM, Wani SE, Visconti MR, Alonso N, Goodman K, Brandi ML, Cundy T, Chung PY, Dargie R, Devogelaer JP, Falchetti A, Fraser WD, Gennari L, Gianfrancesco F, Hooper MJ, Van Hul W, Isaia G, Nicholson GC, Nuti R, Papapoulos S, Montes Jdel P, Ratajczak T, Rea SL, Rendina D, Gonzalez-Sarmiento R, Di Stefano M, Ward LC, Walsh JP, Ralston SH; Genetic Determinants of Paget's Disease (GDPD) Consortium. Genome-wide association identifies three new susceptibility loci for Paget's disease of bone. Nat Genet 2011;43:685-9.
- Gianfrancesco F, Rendina D, Di Stefano M, Mingione A, Esposito T, Merlotti D, Gallone S, Magliocca S, Goode A, Formicola D, Morello G, Layfield R, Frattini A, De Filippo G, Nuti R, Searle M, Strazzullo P, Isaia G, Mossetti G, Gennari L. A nonsynonymous TNFRSF11A variation increases NPκB activity and the severity of Paget's disease. J Bone Miner Res 2012;27:443-52.
- Rendina D, Mossetti G, Soscia E, Sirignano C, Insabato L, Viceconti R, Ignarra R, Salvatore M, Nunziata V. Giant cell tumor and Paget's disease of bone in one family: geographic clustering. Clin Orthop Relat Res 2004;421:218-24.
- Gianfrancesco F, Rendina D, Merlotti D, Esposito T, Amyere M, Formicola D, Muscariello R, De Filippo G, Strazzullo P, Nuti R, Vikkula M, Gennari L. Giant cell tumor occurring in familial Paget's disease of bone: report of clinical characteristics and linkage analysis of a large pedigree. J Bone Miner Res. 2013;28:341-50.
- Wu RK, Trumble TE, Ruwe PA. Familial incidence of Paget's disease and secondary osteogenic sarcoma. A report of three cases from a single family. Clin Orthop Relat Res. 1991;265:306-9.
- Malovannaya A, Lanz RB, Jung SY, Bulynko Y, Le NT, Chan DW, Ding C, Shi Y, Yucer N, Krenciute G, Kim BJ, Li C, Chen R, Li W, Wang Y, O'Malley BW, Qin J. Analysis of the Human Endogenous Coregulator Complexome. Cell 2011;145:787-799.
- Rendina D, Gennari L, De Filippo G, Merlotti D, de Campora E, Fazioli F, Scarano G, Nuti R, Strazzullo P, Mossetti G. Evidence for increased clinical severity of familial and sporadic Paget's disease of bone in Campania, southern Italy. J Bone Miner Res 2006;21:1828-35.
- Gennari L, Gianfrancesco F, Di Stefano M, Rendina D, Merlotti D, Esposito T, Gallone S, Fusco P, Rainero I, Fenoglio P, Mancini M, Martini G, Bergui S, De Filippo G, Isaia G, Strazzullo P, Nuti R, Mossetti G. SQSTM1 gene analysis and gene-environment interaction in Paget's disease of bone. J Bone Miner Res 2010;25:1375-84.
- Michou L, Morissette J, Gagnon ER, Marquis A, Dellabadia M, Brown JP, Siris ES. Novel SQSTM1 mutations in patients with Paget's disease of bone in an unrelated multiethnic American population. Bone 2011;48:456-60.

## Claims

1. An isolated nucleic acid which comprises the whole nucleotide sequence of the ZNF687 gene, wherein said whole nucleotide sequence of the ZNF687 gene comprises at least one mutation with respect to the wild-type nucleotide sequence of the ZNF687 gene as represented by SEQ ID NO:1, **characterized in that** the mutation is selected from the group consisting of:
- C>G at nucleotide position 2810 (SEQ ID NO:4),
- G>T at nucleotide position 725 (SEQ ID NO:2), and
- C>T at nucleotide position 1994 (SEQ ID NO:3).

2. The isolated nucleic acid according to claim 1, comprising the nucleotide sequence SEQ ID NO:2, SEQ ID NO:3 or SEQ ID NO:4.

3. The isolated nucleic acid according to claim 1 or 2, which is a genomic DNA, an mRNA or a cDNA.

4. An oligonucleotide or a probe which comprises from 10 to 30 consecutive nucleotides of the nucleic acid sequence according to any one of claims 1 to 3 and which includes said mutation.

5. An expression vector which comprises a nucleic acid sequence according to any one of claims 1 to 3.

6. A host cell which comprises the expression vector according to claim 5.

7. An *in vitro* or *ex vivo* method for diagnosing Paget's disease of bone or bone tumor associated with Paget's disease or for determining the predisposition of a subject affected by Paget's disease of bone to develop a bone tumor, which comprises the step of determining, in a nucleic acid sample of a subject suspected of being affected by Paget's disease of bone or bone tumor or in a nucleic acid sample of a subject affected by Paget's disease of bone, the presence of at least one mutation in the wild-type nucleotide sequence of the ZNF687 gene as represented by SEQ ID NO:1, the presence of said at least one mutation being indicative of Paget's disease of bone or bone tumor or of a predisposition to develop a bone tumor.

8. The method according to claim 7, wherein said mutation is located in the ZNF687 gene coding region and is preferably a missense mutation.

9. The method according to claim 8, wherein the mutation is a missense mutation selected from the group consisting of:
- C>G at nucleotide position 2810 (SEQ ID NO:4),
- G>T at nucleotide position 725 (SEQ ID NO:2), and
- C>T at nucleotide position 1994 (SEQ ID NO:3).

10. The method according to any one of claims 7 to 9, wherein the mutation is detected by nucleic acid amplification reaction and detection of the amplification product.

11. The method according to any one of claims 7 to 9, wherein the mutation is detected by sequencing the whole nucleotide sequence of the ZNF687 gene or a portion thereof.

12. An isolated mutated ZNF687 protein, **characterized in that** it is encoded by a nucleic acid according to any one of claims 1 to 3, the isolated mutated ZNF687 protein being preferably selected from the group consisting of SEQ ID NO:5, SEQ ID NO:6 and SEQ ID NO: 7.

13. An *in vitro* or *ex vivo* method for diagnosing Paget's disease of bone or bone tumor associated with Paget's disease or for determining the predisposition of a subject affected by Paget's disease of bone to develop a bone tumor, which comprises the step of determining, in a biological sample from a subject suspected of being affected by Paget's disease of bone or bone tumor or in a nucleic acid sample of a subject affected by Paget's disease of bone, the presence of at least one ZNF687 protein which is mutated compared to the wild-type ZNF687 protein sequence as represented by SEQ ID NO:8, the presence of said at least one mutated protein being indicative of Paget's disease of bone or bone tumor or of a predisposition to develop a bone tumor.

14. The method according to claim 13, wherein said bone tumor is a giant cell tumor or an osteosarcoma.

15. The method according to claim 13 or 14, which comprises contacting said biological sample with an antibody which is capable of specifically recognizing an isolated mutated ZNF687 protein according to claim 12, wherein the method is preferably an ELISA assay.

## Patentansprüche

1. Isolierte Nukleinsäure, welche die gesamte Nukleotidsequenz des ZNF687 Gens umfasst, wobei die gesamte Nukleotidsequenz des ZNF687 Gens mindestens eine Mutation in Bezug auf die Wildtyp-Nukleotidsequenz des ZNF687 Gens, wie durch SEQ ID NO:1 dargestellt, umfasst, **dadurch gekennzeichnet, dass** die Mutation ausgewählt ist aus der Gruppe, bestehend aus:
- C>G an Nukleotidposition 2810 (SEQ 10 NO:4),
- G>T an Nukleotidposition 725 (SEQ ID NO:2), und
- C>T an Nukleotidposition 1994 (SEQ ID NO:3).

2. Isolierte Nukleinsäure nach Anspruch 1, umfassend die Nukleotidsequenz SEQ ID NO:2, SEQ ID NO:3 oder SEQ ID NO:4.

3. Isolierte Nukleinsäure nach Anspruch 1 oder 2, die eine genomische DNA, eine mRNA oder eine cDNA ist.

4. Oligonukleotid oder eine Sonde, die 10 bis 30 aufeinanderfolgende Nukleotide der Nukleinsäuresequenz nach einem der Ansprüche 1 bis 3 umfasst und die Mutation beinhaltet.

5. Expressionsvektor, der eine Nukleinsäuresequenz nach einem der Ansprüche 1 bis 3 umfasst.

6. Wirtszelle, die den Expressionsvektor nach Anspruch 5 umfasst.

7. *In vitro* oder *ex vivo* Verfahren zur Diagnose von Morbus Paget von Knochen oder Knochentumor in Zusammenhang mit Morbus Paget oder zum Bestimmen der Prädisposition eines Subjekts, das von Morbus Paget von Knochen betroffen ist, einen Knochentumor zu entwickeln, das einen Schritt des Bestimmens der Anwesenheit von mindestens einer Mutation in der Wildtyp-Nukleotidsequenz des ZNF687 Gens, wie durch SEQ ID NO:1 dargestellt, in einer Nukleinsäureprobe eines Subjekts, das im Verdacht steht von Morbus Paget von Knochen oder Knochentumor betroffen zu sein, oder in einer Nukleinsäureprobe eines Subjekts, das von Morbus Paget von Knochen betroffen ist, wobei die Anwesenheit der mindestens einen Mutation auf Morbus Paget von Knochen oder Knochentumor oder auf eine Prädisposition einen Knochentumor zu entwickeln hinweist.

8. Verfahren nach Anspruch 7, wobei die Mutation im kodierenden Bereich des ZNF687 Gens liegt und vorzugsweise eine Missense-Mutation ist.

9. Verfahren nach Anspruch 8, wobei die Mutation eine Missense-Mutation ist, ausgewählt aus der Gruppe, bestehend aus:
- C>G an Nukleotidposition 2810 (SEQ ID NO:4),
- G>T an Nukleotidposition 725 (SEQ ID NO:2), und
- C>T an Nukleotidposition 1994 (SEQ ID NO:3).

10. Verfahren nach einem der Ansprüche 7 bis 9, wobei die Mutation durch Nukleinsäureamplifikationsreaktion und Detektion des amplifizierten Produkts detektiert wird.

11. Verfahren nach einem der Ansprüche 7 bis 9, wobei die Mutation durch Sequenzieren der gesamten Nukleotidsequenz des ZNF687 Gens oder eines Teils davon detektiert wird.

12. Isoliertes mutiertes ZNF687 Protein, **dadurch gekennzeichnet, dass** es von einer Nukleinsäure nach einem der Ansprüche 1 bis 3 kodiert ist, wobei das isolierte mutierte ZNF687 Protein vorzugsweise aus der Gruppe bestehend aus SEQ ID NO:5, SEQ ID NO:6 und SEQ ID NO:7 ausgewählt ist.

13. *In vitro* oder *ex vivo* Verfahren zur Diagnose von Morbus Paget von Knochen oder Knochentumor in Zusammenhang mit Morbus Paget oder zum Bestimmen der Prädisposition eines Subjekts, das von Morbus Paget von Knochen betroffen ist, einen Knochentumor zu entwickeln, das einen Schritt des Bestimmens der Anwesenheit von mindestens einem ZNF687 Protein, das im Vergleich zu der Wildtyp ZNF687 Proteinsequenz, wie durch SEQ ID NO:8 dargestellt, mutiert ist, in einer biologischen Probe eines Subjekts, das im Verdacht steht von Morbus Paget von Knochen oder Knochentumor betroffen zu sein, oder in einer Nukleinsäureprobe eines Subjekts, das von Morbus Paget von Knochen oder Knochentumor betroffen ist, wobei die Anwesenheit des mindestens einen mutierten Proteins auf Morbus Paget von Knochen oder Knochentumor oder auf eine Prädisposition einen Knochentumor zu entwickeln hinweist.

14. Verfahren nach Anspruch 13, wobei der Knochentumor ein Riesenzelltumor oder ein Osteosarkom ist.

15. Verfahren nach Anspruch 13 oder 14, welches das in Kontakt bringen der biologischen Probe mit einem Antikörper umfasst, der in der Lage ist ein isoliertes mutiertes ZNF687 Protein nach Anspruch 12 spezifisch zu erkennen, wobei das Verfahren vorzugsweise ein ELISA Assay ist.

## Revendications

1. Acide nucléique isolé qui comprend la séquence de nucléotides complète du gène ZNF687, dans lequel ladite séquence de nucléotides complète du gène ZNF687 comprend au moins une mutation par rapport à la séquence de nucléotides de type sauvage du gène ZNF687 telle que représentée par SEQ ID N° : 1, **caractérisé en ce que** la mutation est sélectionnée à partir du groupe constitué par :
- C > G dans la position de nucléotide 2810 (SEQ ID N° : 4),
- G > T dans la position de nucléotide 725 (SEQ ID N° : 2), et
- C > T dans la position de nucléotide 1994 (SEQ ID N° : 3).

2. Acide nucléique isolé selon la revendication 1, comprenant la séquence de nucléotides SEQ ID N° : 2, SEQ ID N° : 3 ou SEQ ID N° : 4.

3. Acide nucléique isolé selon la revendication 1 ou 2, qui est un ADN génomique, un ARNm ou un ADNc.

4. Oligonucléotide ou sonde qui comprend de 10 à 30 nucléotides consécutifs de la séquence d'acide nucléique selon l'une quelconque des revendications 1 à 3 et qui inclut ladite mutation.

5. Vecteur d'expression qui comprend une séquence d'acide nucléique selon l'une quelconque des revendications 1 à 3.

6. Cellule hôte qui comprend le vecteur d'expression selon la revendication 5.

7. Procédé *in vitro* ou *ex vivo* pour diagnostiquer la maladie osseuse de Paget ou une tumeur osseuse associée à la maladie de Paget ou pour déterminer la prédisposition d'un sujet atteint de la maladie osseuse de Paget à développer une tumeur osseuse, qui comprend l'étape consistant à déterminer, dans un échantillon d'acide nucléique d'un sujet soupçonné d'être atteint de la maladie osseuse de Paget ou d'une tumeur osseuse ou dans un échantillon d'acide nucléique d'un sujet atteint de la maladie osseuse de Paget, la présence d'au moins une mutation dans la séquence de nucléotides de type sauvage du gène ZNF687 telle que représentée par SEQ ID N° : 1, la présence de ladite au moins une mutation étant indicative de la maladie osseuse de Paget ou d'une tumeur osseuse ou d'une prédisposition à développer une tumeur osseuse.

8. Procédé selon la revendication 7, dans lequel ladite mutation est située dans la région codant pour le gène ZNF687 et est de préférence une mutation faux-sens.

9. Procédé selon la revendication 8, dans lequel la mutation est une mutation faux-sens sélectionnée à partir du groupe constitué par :
- C > G dans la position de nucléotide 2810 (SEQ ID N° : 4),
- G > T dans la position de nucléotide 725 (SEQ ID N° : 2), et
- C > T dans la position de nucléotide 1994 (SEQ ID N° : 3).

10. Procédé selon l'une quelconque des revendications 7 à 9, dans lequel la mutation est détectée par une réaction d'amplification d'acide nucléique et par une détection du produit d'amplification.

11. Procédé selon l'une quelconque des revendications 7 à 9, dans lequel la mutation est détectée par le séquençage de la séquence de nucléotides complète du gène ZNF687 ou d'une portion de celle-ci.

12. Protéine de ZNF687 mutée isolée, **caractérisée en ce qu'**elle est codée par un acide nucléique selon l'une quelconque des revendications 1 à 3, la protéine de ZNF687 mutée isolée étant de préférence sélectionnée à partir du groupe constitué par SEQ ID N° : 5, SEQ ID N° : 6 et SEQ ID N° : 7.

13. Procédé *in vivo* ou *ex vivo* pour diagnostiquer la maladie osseuse de Paget ou une tumeur osseuse associée à la maladie de Paget ou pour déterminer la prédisposition d'un sujet atteint de la maladie osseuse de Paget à développer une tumeur osseuse, qui comprend l'étape consistant à déterminer, dans un échantillon biologique issu d'un sujet soupçonné d'être atteint de la maladie osseuse de Paget ou d'une tumeur osseuse ou dans un échantillon d'acide nucléique d'un sujet atteint de la maladie osseuse de Paget, la présence d'au moins une protéine de ZNF687 qui est mutée par rapport à la séquence de protéine de ZNF687 de type sauvage telle que représentée par SEQ ID N° : 8, la présence de ladite au moins une protéine mutée étant indicative de la maladie osseuse de Paget ou d'une tumeur osseuse ou d'une prédisposition à développer une tumeur osseuse.

14. Procédé selon la revendication 13, dans lequel ladite tumeur osseuse est une tumeur à cellules géantes ou un ostéosarcome.

15. Procédé selon la revendication 13 ou 14, qui comprend la mise en contact dudit échantillon biologique avec un anticorps qui est capable de reconnaître spécifiquement une protéine de ZNF687 mutée isolée selon la revendication 12, dans lequel le procédé est de préférence un dosage ELISA.
